(19) Europäisches Patentamt
European Patent Office
Office européen des brevets

(11) EP 3 102 637 B1

(12) **EUROPEAN PATENT SPECIFICATION**

(45) Date of publication and mention of the grant of the patent:
**11.04.2018 Bulletin 2018/15**

(21) Application number: **15704492.6**

(22) Date of filing: **04.02.2015**

(51) Int Cl.:
*C09B 69/10* (2006.01) *C09B 67/02* (2006.01)
*C07K 1/22* (2006.01) *C07K 16/00* (2006.01)

(86) International application number:
**PCT/EP2015/052246**

(87) International publication number:
**WO 2015/117984 (13.08.2015 Gazette 2015/32)**

(54) **METHOD FOR PURIFICATION OF ANTIBODIES, ANTIBODY FRAGMENTS OR ENGINEERED VARIANTS THEREOF USING SPECIFIC ANTHRAQUINONE DYE-LIGAND STRUCTURES**

REINIGUNGSVERFAHREN FÜR ANTIKÖRPER, ANTIKÖRPERFRAGMENTE ODER MANIPULIERTE VARIANTEN DAVON MIT SPEZIFISCHEN ANTHRACHINON-FARBSTOFFLIGANDENSTRUKTUREN

PROCÉDÉ DE PURIFICATION D'ANTICORPS, FRAGMENTS D'ANTICORPS OU LEURS VARIANTS MANIPULÉS À L'AIDE DE STRUCTURES LIGANDS COLORANTS D'ANTHRAQUINONE SPÉCIFIQUES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **04.02.2014 IN 324DE2014**
**13.03.2014 EP 14159349**

(43) Date of publication of application:
**14.12.2016 Bulletin 2016/50**

(73) Proprietor: **BASF SE**
**67056 Ludwigshafen am Rhein (DE)**

(72) Inventors:
- **FELLE, Max Fabian**
  **67346 Speyer (DE)**
- **GRANSTRÖM, Mari**
  **FI-00120 Helsinki (FI)**
- **LINDER, David**
  **79108 Freiburg (DE)**
- **KORDE, Shilpa S.**
  **Mumbai 400016 (IN)**
- **SAMEL, Amarish**
  **Mumbai 400016 (IN)**

(56) References cited:
**WO-A1-2004/019009 WO-A1-2009/053360**
**WO-A1-2010/102114 WO-A2-2006/108760**
**US-A- 4 016 149 US-A- 4 043 997**

- **"GE Healthcare Life Sciences Instructions 71-7055-00 AI Affinity chromatography", , 1 February 2011 (2011-02-01), XP055135727, Retrieved from the Internet: URL:https://www.gelifesciences.com/gehcls_images/GELS/Related Content/Files/1314823637792/litdoc71705500AI_20110831230129.pdf [retrieved on 2014-08-21]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

The present invention relates to novel adsorbents applicable a process for the separation or purification of antibodies, antibody fragments or engineered variants thereof, which comprise anthraquinone dye ligands; corresponding purification processes; and corresponding analytical or preparative separation kits.

## Background of the invention:

Reactive dyes are well established products which are widely used in the textile industry for the coloration of cellulosic textiles, such as cotton or viscose. The molecule of such a reactive dye may be viewed as being made up of two interlinked units, each of which has a distinct function. One unit is the chromophore whose function is to impart the desired color and other tinctorial properties to the textile. The other unit is the fibre reactive group which, under well established application conditions, reacts chemically with cellulose to covalently bind the dye molecules to the textile to produce a dyed material which is highly resistant to washing processes.

Whilst reactive dyes were initially developed to solve certain problems encountered in the dyeing and printing of cellulosic textile materials, they have, over the years, been found to possess properties, which make them of use outside the textile dyeing field.

It is known, for example, from U.S. Pat. No's. 4,016,149 and 4,546,161, that these dyes can be attached by similar techniques to carbohydrate substrates, such as polymers and co-polymers derived from agarose, dextrose, dextrans, etc. Reaction products of such carbohydrate substrates and certain commercially available reactive dyes have been used as adsorbents for the chromatographic separation or purification of certain proteinaceous materials. Among the commercial textile dyes, which have been used as ligands for protein or enzyme separation, are certain blue anthraquinone dyes comprising a mono chloro triazine moiety, such as C.I. Reactive Blue 2 (Cibacron Blue 3GA), C.I. Reactive Blue 5 and C.I. Reactive Blue 49. Generally, these adsorbents comprise more than one functionality, such as hydrophobic and ionic or hydrophilic interactions and are often referred to as mixed-mode chromatography (MMC) adsorbents. In contrast to ion exchange (IEX) or hydrophobic interaction (HIC) chromatography with one defined mode-of-interaction of protein and ligand (charge and hydrophobicity of the protein respectively), the interaction of a protein with a mixed-mode ligand is a complex, multi-factorial interaction dependent on both charge and hydrophobicity distribution of the protein at a given pH and solvent and the structural arrangement of functional groups of the ligand.

Affinity chromatography is based on the differential strong affinities of the proteins or enzymes for the ligands on the stationary phase of the adsorbent and is increasingly proving to be very crucial in high-end application areas like biopharmaceuticals and diagnostics as described, for example, in WO 2004/052870. The nature of the interaction may be hydrogen-bonding, electrostatic forces, stacking as a result of favorable geometry or any other aspect that encourages the ligand-target relationship. The ligand-target interaction should be sufficiently strong to allow the removal of the other contaminant molecules from a mixture while keeping the ligand-target complex intact, i.e. the proteins that have affinity to the ligands bind to the matrix while the others with no affinity are washed out. The bound proteins can be selectively eluted under conditions of varying pH, ionic strength and buffers as described, for example, in P.D.G. Dean, W.S. Johnson and F.A. Middle (Ed), Affinity Chromatography: A Practical Approach, 1982. The protein mixture can be subjected to separation by shaking or passing over a column with the adsorbent.

Immunoglobulins are glycoproteins consisting of at least one Y-shaped building block having two identical light chains (of weight ~25 kDa) and two identical heavy chains (of weight ~50 kDa). Each chain is composed of constant and variable regions which are divided into individual domains. Five distinct classes of immunoglobulin (corresponding to certain heavy chain isotypes $\alpha$ (IgA), $\delta$ (IgD), $\varepsilon$ (IgE), $\mu$ (IgM) and $\gamma$ (IgG)) are observed in higher mammals. These molecules differ in size, charge, biological properties, amino acid composition and carbohydrate content. The light chains may be present in one out of two isotypes, termed kappa and lambda.

Applicability of immunoglobulins in the field of diagnostics or therapeutics, or preparative and analytical applications is well established. In contrast to polyclonal antibodies, monoclonal antibodies (Mabs) are antibodies that have identical specificity towards a single antigen and constitute the fastest growing sector in the biopharmaceutical industry. Besides the use of specific monoclonal antibodies as biopharmaceuticals, there is also an increasing interest in the use of different types of antibody fragments or engineered protein variants derived therefrom, since they may differ both chemically and functionally from a polyclonal immunoglobulin preparation or monoclonal antibodies.

The group of fragment antibodies (fAbs) encompasses fragments obtainable from intact, in particular monoclonal antibodies, which fragments retain antigen binding properties while avoiding disadvantages associated with the intact whole immunoglobulin molecule. In particular, such fragments offer a number of advantages over the intact protein for imaging and therapy. eThey have fewer side-effects in patients due to the absence of the Fc (heavy chain) region or portions of the molecule recognized as foreign by the patient's immune system, and they can be modified to include therapeutic payloads. There are several types of antibody fragments. They are either immunoglobulin fragments prepared by specific endopeptidase enzyme digestion or that have been genetically engineered and are produced by corresponding

recombinant host cells. They may have one or more, identical or different antigen binding sites. fAbs include, for example, monovalent fragments such as Fab', Fab and scFv; bivalent fragments such as $F(ab')_2$ diabodies and minibodies; and multivalent fragments such as triabodies, tetrabodies, and TandAbs.

The most important method of producing fAbs is by recombinant technology. Such techniques use a host cell to express the desired fAb, which is then separated from the production medium and purified. Purification is commonly achieved by chromatography, and is typically a complicated, multi-step process. This complexity inevitably limits the yields of fAb that can be obtained, and significantly slows the manufacturing process.

Many whole antibodies are purified by using Protein A affinity chromatography. However, Protein A is recognized as having a number of deficiencies, including poor stability, denaturation, high cost and regulatory concerns arising from the fact that Protein A is itself biological material. Synthetic affinity ligands have therefore been developed as alternative for the purification of antibodies having an affinity for Protein A. fAbs are not purified by Protein A affinity chromatography, because the fAbs do not have an affinity for Protein A, and therefore do not bind.

WO 2006/108760 of the present applicant describes anthraquinone dye ligands and their use for the separation of biological materials, selected from peptides, polypeptides, proteins, nucleotides, polynucleotides, nucleic acids, steroids, lipids, hormones, and in particular enzymes, proteins and peptides.

Triazine-based dyes for the affinity purification of immunoglobulin molecules are disclosed in WO 97/10887, WO 2004/035199 and WO 2007/099374.

WO 2007/004954 suggests as dyes for the affinity purification of IgG and fragments thereof certain [1,2,4] -triazolo-[1,5-A]pyrimidine derivatives.

WO 2009/138714 also suggests the use of certain triazine dyes for purifying fAbs.

WO 2010/102114 describes the use of blue-dye adsorbents for the purification of fAbs in combination with organic polymers.

As methods which have been suggested for separation of antibodies, antibody fragments or engineered variants thereof so far are still associated with disadvantages, there is a need for improved methods for antibody, like fAb, separation. Moreover, high specific binding capacity is indispensible for application in pharmaceutical purification processes.

## Summary of the invention

The present inventors tested a multitude of derivatives of anthraquinone dye (blue-dye) ligands as adsorbent for the improved separation and purification of antibodies, antibody fragments and variants thereof. Surprisingly, only a subpopulation of anthraquinone dye derivatives (attached to a carrier material) comprising anthraquinone dye derivatives as further defined below provide an improved method of separating and purifying fragments derived from immunoglobulin molecules.

## Brief description of the drawings

Figure 1 illustrates a) the results of analytical sice exclusion chromatography (SEC) and b) SDS-PAGE performed with IgG monomers and aggregates prepared therefrom (by heat treatment as specified in the experimental part).

Figure 2 illustrates (a) the observed chromatographic separation profile for prior art resin Blue Sepharose ® in an experiment separating human polyclonal IgG antibody aggregates (Mu) and monomers (Mo) and the results of (b) SDS-PAGE as well as (c) SEC of pooled fractions A and B of the chromatographic separation (mAU = milli absorption units at 280 nm; CV = column volumes).

Figure 3 illustrates the observed chromatographic separation profile for BASF resins BR36, BR37, BR45, BR46 and BR47 of the invention in an experiment separating human polyclonal IgG antibody aggregates (Mu) and monomers (Mo)

Figure 4 illustrates the results of SDS-PAGE of single fractions of each chromatographic separation as depicted in Figure 2a and Figure 3.

Figure 5 illustrates the results of an analysis of pools A and B (from chromatographic separations of Figure 3 by analytical size exclusion chromatography.

Figure 6 illustrates the results of an analysis of pools A and B (from chromatographic separations) of Figure 3 by

SDS PAGE,

## Detailed description

### a) Particular embodiments:

The present invention relates to the following particular embodiments:

1. An adsorbent for chromatographic purification of immunoglobulin molecules, which adsorbent comprises a carrier matrix (or carrier) to which a dye ligand is covalently attached, wherein said dye ligand is selected from anthraquinone dyes and wherein the ligand density of said adsorbent is more than150, like 151 to 500 or 160 to 500 or 170 to 400 or 200 to 350 or 180 to 350 or 190 to 330 or 220 to 330 or 250 to 300 μmoles/g dry adsorbent, wherein ligand densities in the range of 160 to 500 μmoles/g dry adsorbent being preferred, ligand densities in the range of 170 to 400 μmoles/g dry adsorbent being more preferred, and 190 to 330 μmoles/g dry adsorbent being most preferred.

2. The adsorbent of embodiment 1, wherein said adsorbent comprises the reaction product of said carrier matrix (as for example chemically activated; in particular chemically activated in a manner known per se in the art, and as for example described in the experimental part), and an anthraquinone compound of the general formula 1

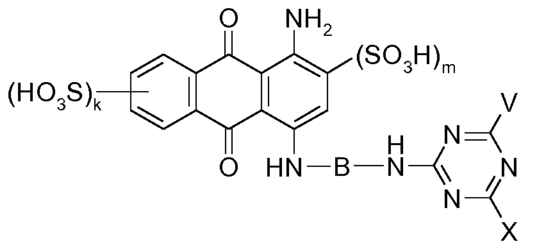

(1)

wherein

| | |
|---|---|
| X | is halogen, in particular F, Cl or Br; via which group the formation of said covalent attachment to the carrier is effected by chemical reaction; |
| k and m | are each independently of each other 0 or 1 and the sum of k+m being 1 or 2; in particular k is 0 and m is 1; |
| B | is an aromatic radical, comprising one or more, condensed or non-condensed, in particular one, optionally mono- or polysubstituted aromatic rings; |
| V | is a substituent of formula |

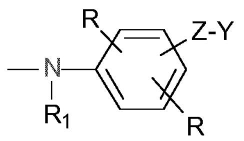

(2a)

or

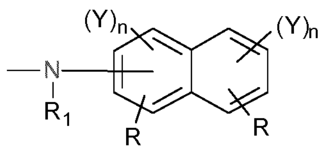

(2b)

like

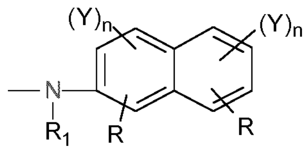

(2b')

or

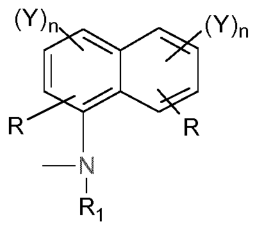

(2b'')

wherein

- n is each 0 or 1; with the proviso that both n are not simultaneously 0;
- R are the same or different and are selected from H, alkyl, $NH_2$, NH-alkyl, NH-aryl, $N(alkyl)_2$, $NO_2$, OH, O-alkyl, CN, C(O)alkyl, C(O)aryl, $C(O)NH_2$, C(O)N(H)alkyl, $C(O)N(alkyl)_2$, C(O)N(H)aryl, or halogen; in particular R is hydrogen; alky in any of the above meanings is in particular $C_1$-$C_4$alkyl; and aryl is in particular phenyl;
- $R_1$ is hydrogen or optionally substituted $C_1$-$C_4$alkyl, in particular $R_1$ is hydrogen;
- Z is selected from a chemical bond, $-(CH_2)_{n1}-$ with n1 being an integer from 1 to 4, arylene, $-CH_2$-arylene-, $-SO_2$-arylene-, -C(O)N(H)arylene-, $-SO_2N(H)$arylene-, -CH=CH-, $-SO_2-CH_2-CH_2-$; $-C(O)NH-(CH_2)_{n2}-$ with n2 being an integer from 1 to 4; in particular, Z is a chemical bind or a $-SO_2-CH_2-CH_2-$ group; arylene in any of the above meanings is in particular phenylene; and
- Y are the same or different polar functional groups selected from $-SO_3H$,$-OSO_3H$, $-CO_2H$, $-P(O)(OH)_2$, $-OP(O)(OH)_2$, OH and SH; in particular Y is selected from $-SO_3H$, $-OSO_3H$, and $-CO_2H$;

and optionally a linker molecule forming a, preferably covalent, linking group between carrier matrix and anthraquinone compound.

Particular examples are compounds of formula (1) wherein B is an optionally substituted phenylene bridge wherein said N-groups are linked to the aromatic ring B in meta or para position.

Further particular examples are compounds of formula (1) wherein V is a substituent of formula (2a), in particular, of the formula

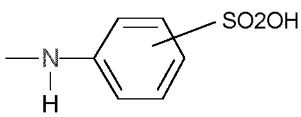

(2a')

like

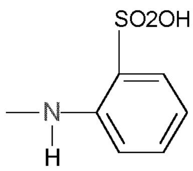

(2a")

Preferred adsorbents comprise a carrier matrix to which at least one of the above-mentioned particular dyes of this embodiment is covalently attached, wherein preferred ligand densities are in the range of 160 to 500 μmoles/g dry adsorbent, more preferred ligand densities are in the range of 1700 to 400 μmoles/g dry adsorbent, and most preferably in the range of 190 to 330 μmoles/g dry adsorbent.

3. The adsorbent of anyone of the preceding embodiments, wherein
B is is a substituent of formula

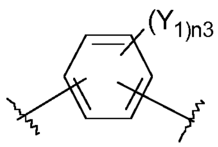

(3a)

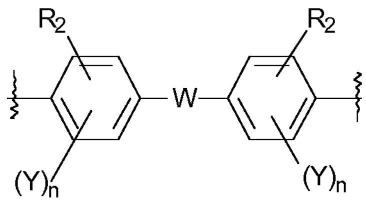

(3b)

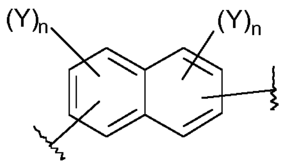

(3c)

or

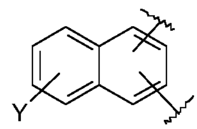

(3d)

wherein

Y is as defined above,
n is 0 ir 1
n3 is 0, 1, 2, 3 or 4;
$Y_1$ are the same or different and are selected from halogen, in particular Cl, alkyl, in particular $C_1$-$C_4$alkyl, alkoxy, in particular $C_1$-$C_4$alkoxy, $NO_2$ or Y, wherein Y is as defined above;
W is a chemical bond or is selected from -C(O)N(H)-, -$SO_2$N(H)-, -$SO_2$- or -N(H)-; and
$R_2$ is selected from H or alky, in particular $C_1$-$C_4$alkyl.

Particular examples are compounds of formula (1) wherein B is an optionally substituted phenylene bridge of the formula 3a linked to said N-groups in meta or para position.
Further particular examples are compounds of formula (1) wherein B is a phenylene bridge of the formula 3a linked

to said N-groups in meta or para position, and wherein n3 is 1, 2, 3 or 4, and wherein at least one $Y_1$ radical is Y, in particular $-SO_3H$, $-OSO_3H$, or $-CO_2H$. For example, n3 is 0 or 1 and $Y_1$ is-$SO_3H$; or N3 is 4 and one $Y_1$ is $-SO_3H$ and the other three residues are identical or different $C_1$-$C_4$alkyl, like methyl.
Further preferred adsorbents comprise a carrier matrix to which at least one of the above-mentioned particular dyes of this embodiment is covalently attached, wherein preferred ligand densities are in the range of 160 to 500 μmoles/g dry adsorbent, more preferred ligand densities are in the range of 170 to 400 μmoles/g dry adsorbent, and most preferably in the range of 190 to 330 μmoles/g dry adsorbent.

4. The adsorbent of anyone of the preceding embodiments, wherein B is an aromatic radical of formula 3a.
Further preferred adsorbents comprise a carrier matrix to which at least one of the above-mentioned particular dyes of this embodiment is covalently attached, wherein preferred ligand densities are in the range of 160 to 500 μmoles/g dry adsorbent, more preferred ligand densities are in the range of 170 to 400 μmoles/g dry adsorbent, and most preferably in the range of 190 to 330 μmoles/g dry adsorbent.

5. The adsorbent of anyone of the preceding embodiments, wherein V is a substituent for formula 2a, like 2a' or 2a".
Further preferred adsorbents comprise a carrier matrix to which at least one of the above-mentioned particular dyes of this embodiment is covalently attached, wherein preferred ligand densities are in the range of 160 to 500 μmoles/g dry adsorbent, more preferred ligand densities are in the range of 170 to 400 μmoles/g dry adsorbent, and most preferably in the range of 190 to 330 μmoles/g dry adsorbent.

6. The adsorbent of embodiment 4, wherein B is an aromatic radical of formula 3a, wherein n3 is 0, 1, 2, 3 or 4 and $Y_1$ is alkyl, like $C_1$-$C_4$alkyl, or Y, like, $-SO_3H$. For example, B may be a 1,3-phenylene group, carrying in positions 2, 4, and 5 a $C_1$-$C_4$alkyl group, in particular methyl group, and in position 5 a residue Y, like $-SO_3H$.
Further preferred adsorbents comprise a carrier matrix to which at least one of the above-mentioned particular dyes of this embodiment is covalently attached, wherein preferred ligand densities are in the range of 160 to 500 μmoles/g dry adsorbent, more preferred ligand densities are in the range of 170 to 400 μmoles/g dry adsorbent, and most preferably in the range of 190 to 330 μmoles/g dry adsorbent.

7. The adsorbent of embodiment 5 wherein V is a substituent for Formula 2a, wherein R is H and Z is selected from a chemical bond or $-SO_2-CH_2-CH_2-$; in particular Z is a chemical bond and Y is $-SO_3H$, $-OSO_3H$, or $-CO_2H$, like $-SO_3H$.; in particular a substituent of Formula 2a' or 2a".
Further preferred adsorbents comprise a carrier matrix to which at least one of the above-mentioned particular dyes of this embodiment is covalently attached, wherein preferred ligand densities are in the range of 160 to 500 μmoles/g dry adsorbent, more preferred ligand densities are in the range of 170 to 400 μmoles/g dry adsorbent, and most preferably in the range of 190 to 330 μmoles/g dry adsorbent.

8. The adsorbent of anyone of the preceding embodiments wherein k is 0 and m is 1.
Further preferred adsorbents comprise a carrier matrix to which at least one of the above-mentioned particular dyes of this embodiment is covalently attached, wherein preferred ligand densities are in the range of 160 to 500 μmoles/g dry adsorbent, more preferred ligand densities are in the range of 170 to 400 μmoles/g dry adsorbent, and most preferably in the range of 190 to 330 μmoles/g dry adsorbent

9. The adsorbent of anyone of the preceding embodiments, wherein said adsorbent comprises said anthraquinone compound attached to said carrier matrix via a linker, wherein said linker is selected from 6-diaminohexane (DAH) 1,4-diaminobutane (DAB) 1,2-diaminoethane (DAE) 1,8-diaminooctane (DAO), 1,10-diaminodecan, and, in particular, tri-2'-aminoethylamine (TREN).
Further preferred adsorbents of this embodiment are those, wherein preferred ligand densities are in the range of 160 to 500 μmoles/g dry adsorbent, more preferred ligand densities are in the range of 170 to 400 μmoles/g dry adsorbent, and most preferably in the range of 190 to 330 μmoles/g dry adsorbent.

10. The adsorbent of anyone of the embodiments 1 to 9, having a mean particle size in the range of 10 to 200, like 20 to 180 or 45 to 165 or 80 to 120 or 90 to 110 μm, determined as D(0,5) or D(4,3), in particular D(4,3).
Further preferred adsorbents of this embodiment are those, wherein preferred ligand densities are in the range of 160 to 500 μmoles/g dry adsorbent, more preferred ligand densities are in the range of 170 to 400 μmoles/g dry adsorbent, and most preferably in the range of 190 to 330 μmoles/g dry adsorbent.

11. A process for chromatographic purification of immunoglobulin molecules, which process comprises contacting a sample containing at least one desired type of immunoglobulin molecules in admixture with contaminating inorganic

and/or organic substances with an adsorbent of anyone of the embodiments 1 to 10, adsorbing said desired type of immunoglobulin molecules on said adsorbent, optionally washing said adsorbent with a washing liquid, and afterwards eluting the adsorbed material in order to obtain an eluate containing said desired immunoglobulin molecules in enriched (i.e. more pure) form.

12. A process for chromatographic purification of immunoglobulin molecules, which process comprises contacting a sample containing at least one desired type of immunoglobulin molecules in a mixture with contaminating inorganic and/or organic substances with an adsorbent of anyone of the embodiments 1 to 10, adsorbing (optionally under selected conditions) said contaminating inorganic and/or organic substances, and obtaining a flow through containing said desired immunoglobulin molecules in a more pure form.
Processes of embodiment 11 and 12 may also be repeatedly performed, they may also be combined in any order and the combined processes may be repeated in any order.

13. The process of embodiments 11 or 12, wherein said desired type of immunoglobulin molecule is a functional antibody (Ab) or fragment antibody (fAb)

14. The process of one of the embodiments 11 to 13, wherein Abs and fAbs are selected from chemically, enzymatically or recombinantly produced Abs or fAbs.

15. The process of embodiment 14, wherein said fAb is selected from the group of Fab, $F(ab')_2$ (or $Fab_2$), $Fab_3$, scFv, bis-scFv, minibody, diabody, triabody, tetrabody, tandab; and single antibody domains and fragments thereof; wherein polyvalent fragments thereof may have the same or different antigen specificity.

16. The process of embodiment 15, wherein said antibody domain is selected from $V_H$, $V_L$, VhH and V-NAR domains.

17. The process of embodiment 16, wherein said domain fragment comprises at least one CDR, in particular at least one of CDR1, CDR2 and CDR3.

18. The process of one of the embodiments 11 to 17, wherein the immunoglobulin containing sample to be purified is contaminated with denatured and/or agglomerated /mulitimerized immunoglobulins, immunoglobulin or antibody degradation products, free light or heavy chains of antibodies, other bio molecules like DNA or host cell proteins.

19. The process of any one of the preceding embodiments 11 to 18, wherein said Ab or fAb is bound to the adsorbent under at pH 4 to 8.

20. The process of on of the embodiments 11 to 19, wherein said sample is contacted repeatedly with identical or different adsorbents as defined in one of the embodiments 1 to 10.

21. The use of a compound as defined in any one of the embodiments 1 to 9 for the separation of immunoglobulin molecules, in particular Abs and fAbs.

22. The use of embodiment 21, wherein said separation is performed for the purpose of removal or the qualitative or quantitative isolation, qualitative or quantitative detection, or characterization of at least one Ab or fAb.

23. A preparative or analytical kit comprising at least one adsorbent carrying an anthraquinone compound as defined in any one of the embodiments 1 to 10, and additional material for identifying at least one separated fAb.

24. A method of preparing an adsorbent of one of the embodiments 1 to 10, which method comprises reacting an optionally activated carrier with a suitable linker, as for example tri-2'-aminoethylamine (TREN), followed by reacting said thus obtained functionalized adsorbent with an anthraquinone compound of embodiment 1 to 9.

**b) Particular definitions**

The terms "separation" and "purification" have the same meaning in the context of the present invention and are used to describe the treatment of a composition comprising a complex mixture proteinaceous material and/or other biological material of different molecular weight and type (like material typically forming cell constituents), which material, contains as one constituent at least one fAb molecule as target and which purification/separation results in a product which contains said target molecule in enriched form.

The term "enriched" means a partial or complete depletion of material other than said target molecule from said complex mixture, in particular, depletion of proteinaceous and/or other biological material as mentioned above.

The term "ligand density" defines the molar content of anthraquinone dye molecules (like those of formula (1)) per dry weight (for example as μmoles/g) of the adsorbent (carrier and directly or covalently attached dye ligand). Said ligand density is determined under standard conditions, by drying the adsorbent, in particular by freeze drying in order to remove residual liquid from the material. A dry power is obtained. Residual liquid in a proportion of less than 1wt.-% or more particularly less than 0,1wt.-%, based on the total weight of the adsorbent, may remain. Then, a predetermined amount of said dry adsorbent material is dissolved in an acid, like HCl, such as in 6N HCl, followed by incubation at elevated temperature, like 1h/40°C or 1h/60°C. Thereby, the dye is set free by hydrolytic cleavage. Afterwards the spectrometric determination of the dye content (at $\lambda_{max}$ of the particular dye) is performed. The entire method is further specified in the experimental part herein below. In view of the fact that the liquid content of adsorbents is relatively high (for commercial Blue Sepharose products liquid contents in the range of about 60 to 90 wt.-%, depending on the method of sample preparation, were observed) expressing a ligand density value per dry weight of said adsorbent is considered best suited to characterize the adsorbent's ligand content. The term "particle size" is defined as mean particle size. Preferably the particles of the present invention a characterized by a narrow, in particular essentially monomodal or monomodal particle size distribution. Particle size determination may be performed in a manner known per se, as for example by applying particle size distribution measurements on a Malvern Mastersizer instrument. Typically the measurement may be performed in 0,1M NaOH. The mean particle size values stated herein are either D(0.5) or D(4.3) values which may slightly differ but which, nevertheless are in the indicated parameter range, D(0.5) represents the mean particle size in μm at which 50% of the distribution is smaller and 50% of the size distribution is larger. D(4,3) represents the volume mean diameter.

As "$C_1$-$C_4$alkyl" in the radical B or V there come into consideration, for example, methyl, ethyl, n-propyl, isopropyl, n-butyl, sec-butyl, tert-butyl or isobutyl, particularly methyl or ethyl, especially methyl.

As "$C_1$-$C_4$alkoxy" or "-O- $C_1$-$C_4$alkyl "in the radical B or V there come into consideration, for example, methoxy, ethoxy, n-propoxy, isopropoxy, n-butoxy, sec-butoxy, tert-butoxy and isobutoxy, particularly methoxy or ethoxy, especially methoxy.

As "halogen" in the radical B or V there come into consideration, for example, fluorine, chlorine and bromine, particularly chlorine or bromine, especially chlorine.

As "arylene" in the radical B or v there come into consideration naphthylen, biphenylen or phenylene, in particular phenylene, and more particular o-, m- and" more particular, p-phenylene.

**c) Particular B and V groups**

In the subsequent Tables 1 and 2 non-limiting examples of groups B and V of formula 1 are listed.

Table 1: Examples of Group B

| No. | Formula | No. | Formula |
|---|---|---|---|
| 1) | 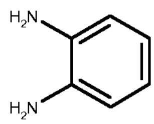 | 9) | 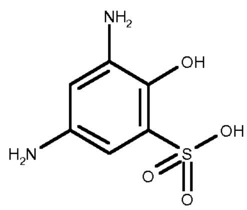 |
| 2) | 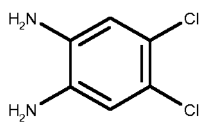 | 10) | 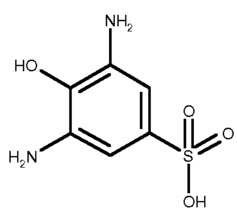 |

(continued)

| No. | Formula | No. | Formula |
|---|---|---|---|
| 3) | 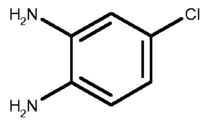 | 11) | 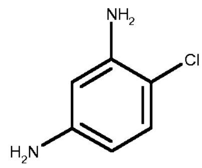 |
| 4) | 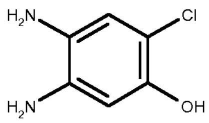 | 12) | 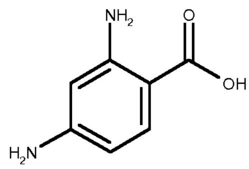 |
| 5) | 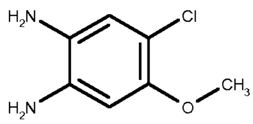 | 13) | 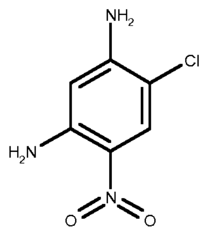 |
| 6) | 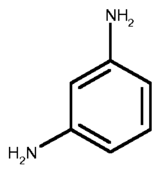 | 14) | 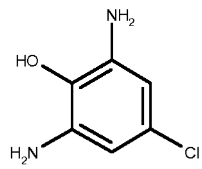 |
| 7) | 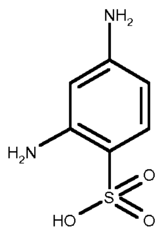 | 15) | 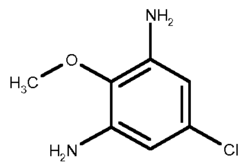 |
| 8) | 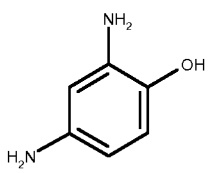 | 16) | 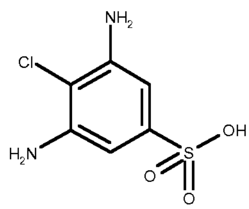 |
| 17) | 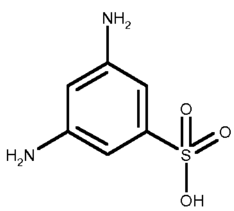 | 25) | 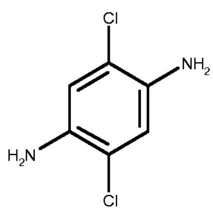 |

(continued)

| No. | Formula | No. | Formula |
|---|---|---|---|
| 18) | 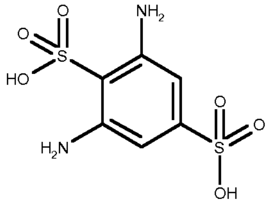 | 26) | 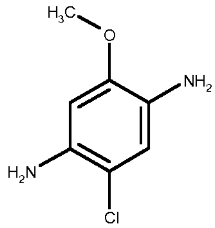 |
| 19) | 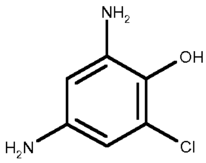 | 27) | 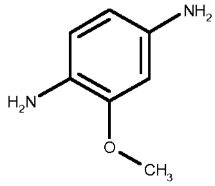 |
| 20) | 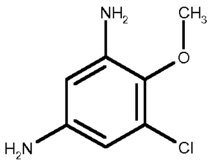 | 28) | 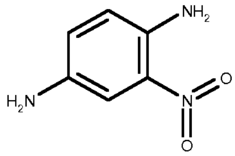 |
| 21) | 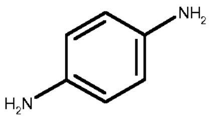 | 29) | 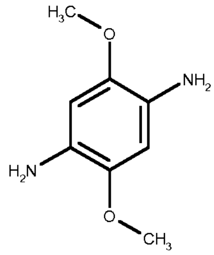 |
| 22) | 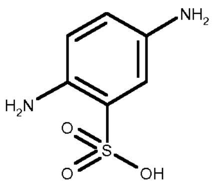 | 30) | 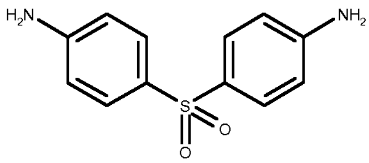 |
| 23) | 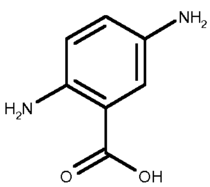 | 31) | 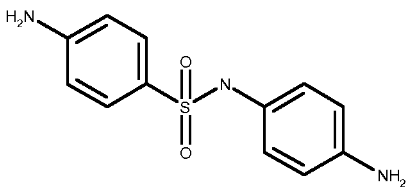 |
| 24) | 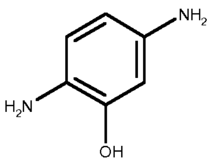 | 32) | 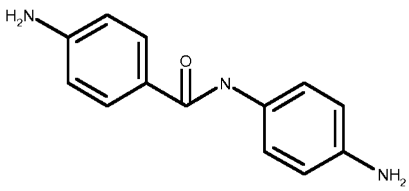 |

(continued)

| No. | Formula | No. | Formula |
|---|---|---|---|
| 33) | 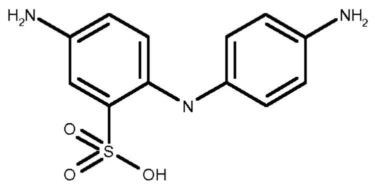 | 40) | 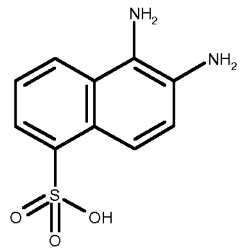 |
| 34) | 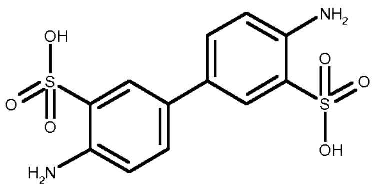 | 41) | 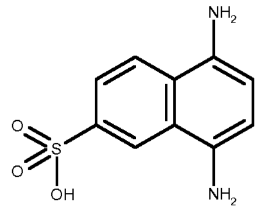 |
| 35) | 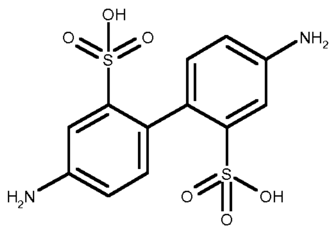 | 42) | 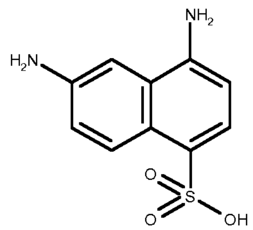 |
| 36) | 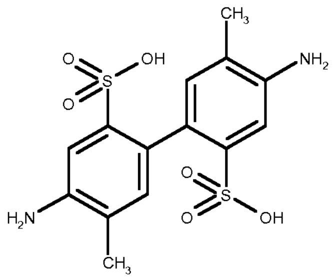 | 43) | 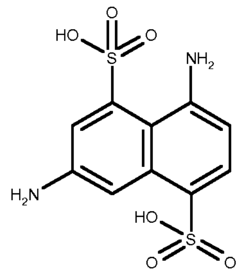 |
| 37) | 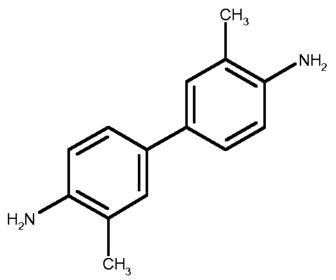 | 44) | 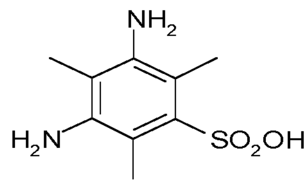 |
| 38) | 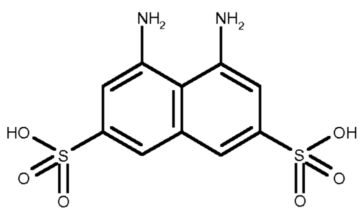 | | |

(continued)

| No. | Formula | No. | Formula |
|---|---|---|---|
| 39) | 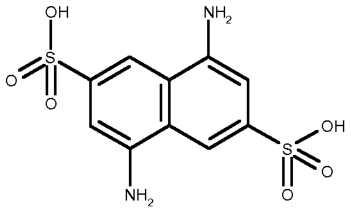 | | |

Table 2: Examples of Group V

| No. | Formula | No. | Formula |
|---|---|---|---|
| 9) | 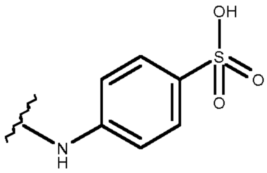 | 17) | 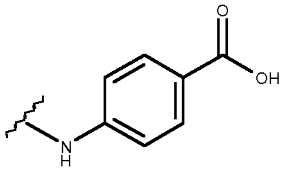 |
| 10) | 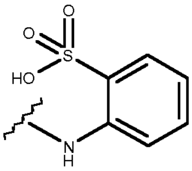 | 18) | 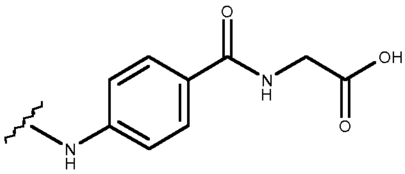 |
| 11) | 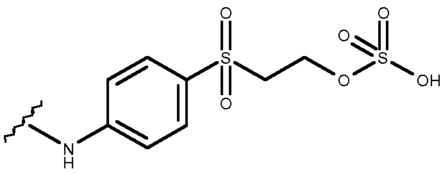 | 19) | 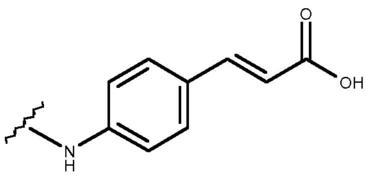 |
| 12) | 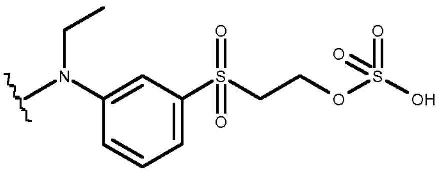 | 20) | 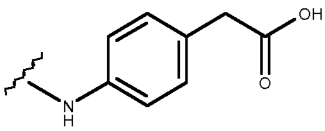 |
| 13) | 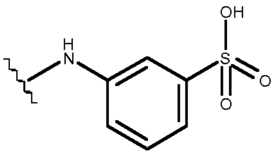 | 21) | 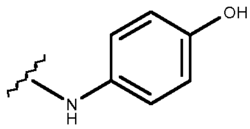 |
| 14) | 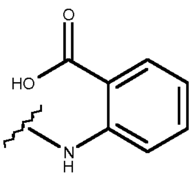 | 22) | 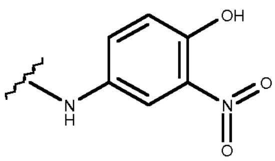 |

(continued)

| No. | Formula | No. | Formula |
|---|---|---|---|
| 15) | 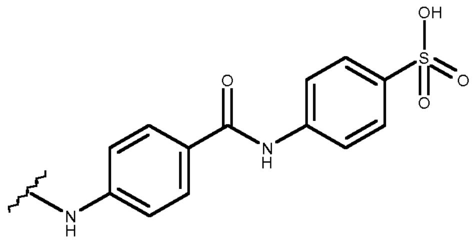 | 23) | 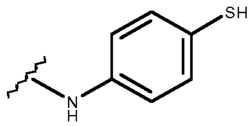 |
| 16) | 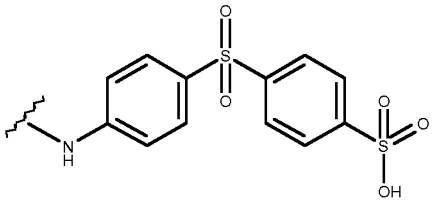 | 24) | 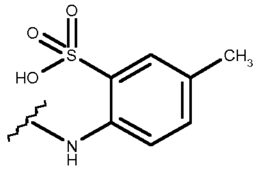 |
| | | | |
| No. | Formula | No. | Formula |
| 17) | 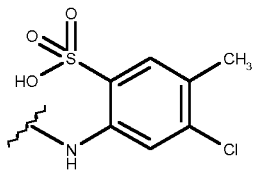 | 28) | 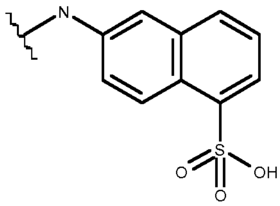 |
| 25) | 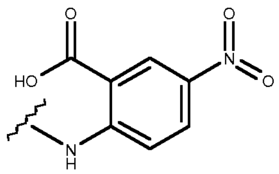 | 29) | 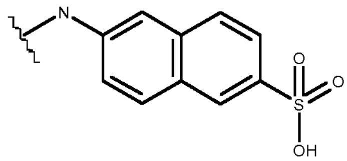 |
| 26) | 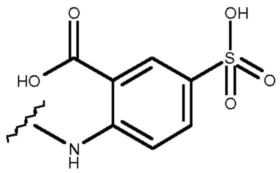 | 30) | 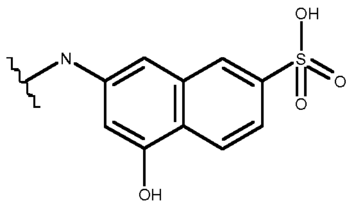 |
| 27) | 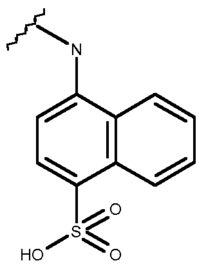 | | |

(continued)

| No. | Formula | No. | Formula |
|---|---|---|---|
| 28) | 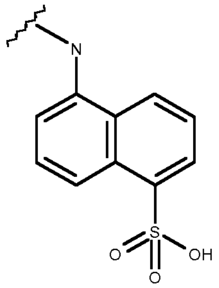 | | |
| 29) | 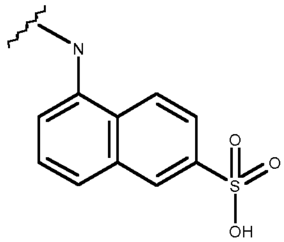 | | |
| 30) | 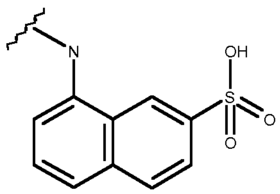 | | |
| 31) | 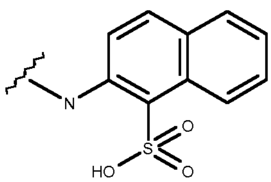 | | |

**d) Particular dye ligands**

In a particular embodiment of the present invention the compound of formula (1) corresponds to a compound of formula

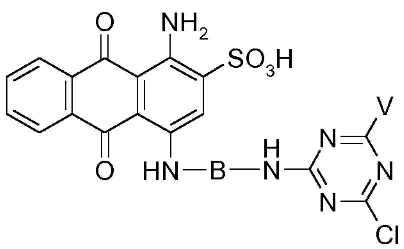

(1a),

wherein

B is a phenylene radical, which is unsubstituted or substituted by sulfo ($-SO_3H$) and optionally further substituted by $C_1$-$C_4$alkyl; as for example a residue of the formula.

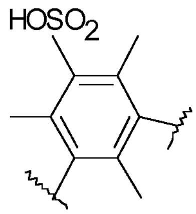

V is phenylamino or N-$C_1$-$C_4$alkyl-N-phenylamino which is substituted in the phenyl ring by sulfo or -$SO_2$-$CH_2$-$CH_2$-O-$SO_2$OH, as for example phenylamino, meta- or para-substituted with -$SO_2$OH;

A structural variation of the compound of formula (1) may account for particular separation or purification problems.

Non-limiting examples of particular compounds of formula 1a are given below in Table 3;

Table 3:

| No | Formula |
|---|---|
| BR1 | 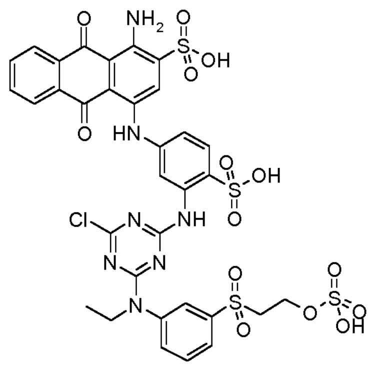 |
| BR6 | 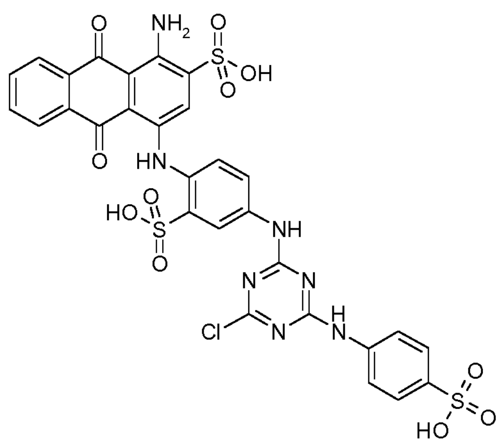 |

(continued)

| No | Formula |
|---|---|
| BR10 | 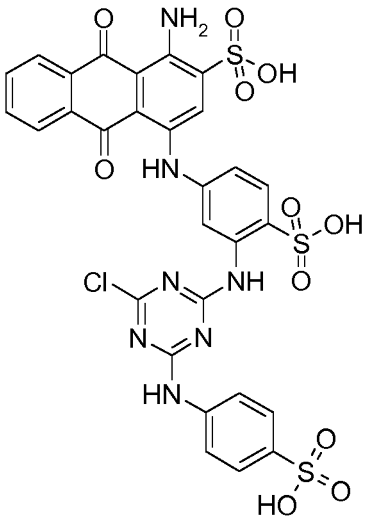 |
| BR11 | 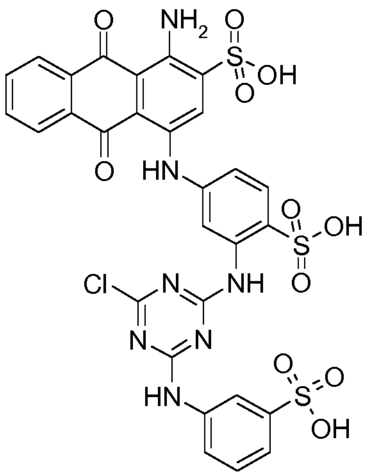 |
| BR15 | 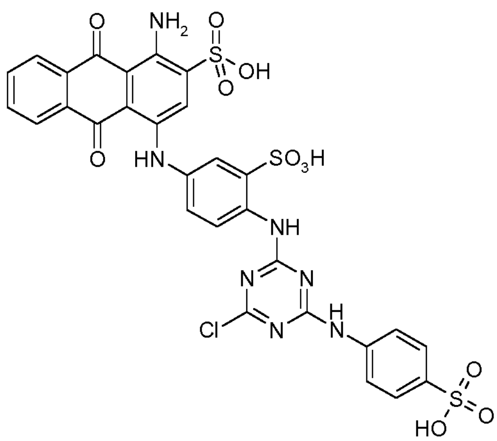 |

(continued)

| No | Formula |
|---|---|
| BR16 | 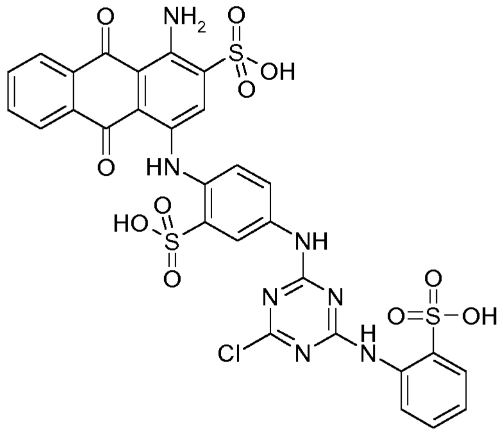 |
| BR20 | 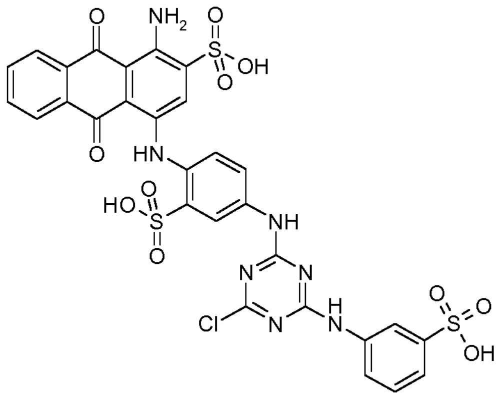 |

**e) Matrix Materials (Substrates and Spacers)**

The substrate, carrier or matrix used to prepare the adsorbents applied in accordance with the present invention may be substantially water-insoluble. As examples of the said substrate there may be mentioned acrylic polymers, such as polyacrylamides or hydroxyalkylmethacrylates, and co-polymers of theses materials, metal oxides, such as zirconia, titania or alumina, silica or porous glass, but the preferred water-insoluble solid support is a polymeric substrate having a plurality of hydroxyl groups to which the compound of formula (1) may become attached through the reactive group. Especially suitable substrates are carbohydrates and modified carbohydrates. Examples of a suitable carbohydrate substrate are agarose, cross-linked agarose, dextrose, dextrans, and modified versions thereof, such as are available as "Sepharose" and "Sephadex" gels ("Sepharose" and "Sephadex" are trade marks of GE Healthcare) and are described in GB 1,540,165. Other polymeric substrates are polyamides. Of particular interest are substrates like agarose and crosslinked agarose.

The adsorbents applied in accordance with the present invention may be prepared by standard techniques, for example, by reacting a compound of formula (1) with a substrate having a group capable of reacting with a reactive group in said compound of formula (1) to form a covalent bond, e.g. a carbohydrate substrate, in the presence of an acid binding agent, such as an alkali metal hydroxide or carbonate, e.g. sodium hydroxide or sodium carbonate, wherein the variables are defined and preferred as given above.

Methods for the preparation of such adsorbents and chromatographic columns containing them are well documented, for example, in U.S. Pat. No's. 4,016,149 and 4,546,161.

As already indicated, the substrate or matrix, for example, agarose, crosslinked agarose, dextrose or dextrans, may be further modified by introduction of spacers of varying length, advantageously, before covalently binding the compound of formula (1) to the spacers. Spacers may allow for an increased interaction of the dye-ligands with the proteinaceous material to be purified, due to an improved accessibility of the dye-ligands, thereby improving the selectivity and the efficiency of the adsorbents. In a particular embodiment, matrices are modified by spacers.

Introduction of spacers is carried out, for example, by treatment of the substrate with epichlorhydrin in the

presence of an acid binding agent, such as an alkali metal hydroxide or carbonate, e.g. sodium hydroxide or sodium carbonate, and subsequently with a polyamine, under suitable temperatures of, for example 20 to 80°C, particularly 20 to 60°C.

Polyamine compounds suitable for the introduction of spacers are either linear or branched, in particular linear spacer groups. They may be selected from the group consisting of aromatic polyamines, aliphatic polyamines and alicyclic polyamines. Typical examples include polyvinyl amine, polyvinyl imine, 1,2-ethylenediamine, hydrazine, hydrazine-2-bis-(3-aminopropyl)-amine, 1,4-diaminocyclohexane, 3-amino-1-methylaminopropane, N-hydroxyethylethylenediamine, N-methyl-bis-(3-aminopropyl)-amine, tetraethylenediamine, 1,4-diaminobutane, 1,6-hexamethylenediamine, 1,8-diaminooctane, tri-2'-aminoethylamine, 1-aminoethyl-1,2-ethylenediamine, diethylenetriamine, tetraethylenepentamine, pentaethylenehexamine, phenylenediamine, toluylenediamine, 2,4,6-triaminotoluene-trihydrochlorides, 1,3,6-triaminonaphthalene, isophoronediamine, xylylenediamine, hydrogenated xylylenediamine, 4,4'-di-diaminophenylmethane, hydrogenated 4,4'-diaminodiphenylmethane, and derivatives of these polyamine monomers; as well as branched spacer, like tri-2'-aminoethylamine.

Aliphatic and/or alicyclic polyamines are of particular interest. Of particular interest are aliphatic and/or alicyclic diamines. There may be mentioned in particular 1,4-diaminobutane, 1,6-hexamethylenediamine, 1,8-diaminooctane and 1,10-diaminodecane.

The polyamines may be used individually or as mixtures of at least two polyamines.

The adsorbent comprising the compound of formula (1) or (1a) bound to a solid support may be in the form of a column for chromatographic separation purposes or in the form of a membrane to allow separation to be carried out in a membrane separation format.

**f) Biological Material for Separation**

The biological material as applied to the adsorbent contains or is suspected to contain immunoglobulin molecules, like antibodies fragment antibody (fAb) material. It may be, for example, in the form of a crude or pre-treated cell culture supernatant, whole cell culture, cell homogenate, or any fraction thereof suitable for applying on an adsorbent. Methods for obtaining suitable proteinaceous sample material are well-known in the art, as for example described in Cooper, Biochemische Arbeitsweisen, Walter de Gruyter, 1981.

fAbs which can be purified by the process of the present invention are sections of antibodies comprising an immunoglobulin domain or an assembly of immunoglobulin domains, which are capable of binding to an antigen, and which, in many embodiments, comprise at least one heavy chain fragment, commonly known as VH domain, or a functional fragment thereof, and/or a light chain fragment, commonly known as VL domain, or a functional fragment thereof, optionally together with at least one other peptide chain, like for example one or more constant domains.

Light chain derived constant domains are commonly designated CL.

Heavy chain derived constant domains are for example: CH1, CH2 and CH3 of an IgG molecule; $C\mu1$, $C\mu2$, $C\mu3$ or $C\mu4$ of an IgM molecule; $C\alpha1$, $C\alpha2$ and $C\alpha3$ of an IgA molecule; $C\varepsilon1$, $C\varepsilon2$, $C\varepsilon3$ or $C\varepsilon4$ of an IgE molecule; or $C\delta1$, $C\delta2$ and $C\delta3$ of an IgD molecule.

In certain embodiments, the fAb comprises a heavy chain derived fragment and a light chain derived fragment, each chain fragment being made up of a constant domain and a variable domain, such fragments being known as a Fab fragment.

In other embodiments, the fAb comprises two or more domains, typically a combination of either the variable and constant domains of either heavy or light chains, combinations of variable domain from two heavy chains, combinations of variable domains from two light chains, or a combination of the variable domain from a light chain and the variable domain from a heavy chain.

In some embodiments, the fAb comprises the VH and VL domains joined by flexible polypeptide linker preventing dissociation (known as single chain Fv, scFv).

In yet further embodiments, the fAb comprises a single domain, or a fragment thereof, typically either the variable heavy chain or a fragment thereof, or the variable light chain or a fragment thereof.

In still further embodiments, the fAb is a multimeric format, such as a bis scFv, Fab2, Fab3, minibody (like scFv-$C_H3$), diabody, triabody, tetrabody (see for example Hollinger et al., Nature Biotechnology, 2005, 23, 9, 1126-1136) or Tandab®.

Examples of fAbs that can be purified by the process of the present invention include protein or polypeptide constructs comprising a combined heavy chain and a light chain, each chain being made up of a constant domain and a variable domain where such immunoglobulin light and heavy chains interact to form a single functional antigen-binding site.

Further examples include VH chain-based domain antibodies, being polypeptides which are capable of binding to a target, the polypeptide comprising at least one binding domain, wherein the binding domain is a single variable domain of a variable heavy chain antibody or a functional fragment thereof.

Yet further examples include VL chain-based domain antibodies, being polypeptides which are capable of binding to a target, the polypeptide comprising at least one binding domain, wherein the binding domain is a single variable domain of a variable light chain antibody or a functional fragment thereof.

The above identified fAbs may be derived from any of the above-identified distinct immunoglobulin classes (IgA, IgD, IgE, IgM or IgG, in particular IgG; and any subclasses thereof (like IgG1, IgG2, IgG3 and IgG4).

Particular fAbs are of the type scFv and Fab.

Immunoglobulin or antibodies to be purified according to the present invention may be of any Ig class (IgA, IgD, IgE, IgM or IgG, in particular IgG; and any subclasses thereof (like IgG1, IgG2, IgG3 and IgG4).

Most preferably, the fAbs or antibodies or engineered variants thereof are produced recombinantly, for example by expression in a host cell, for example in a prokaryotic host such as E. coli or in a eukaryotic host such as Pichia pastoris,or mammalian or plant cells

**g) The separation process**

The separation or purification process according to the present invention may also be carried out by treating, e.g. shaking, the biological material in the presence of the adsorbent comprising the compound of formula (1) in an aqueous environment of suitable pH and ionic strength, separating the adsorbent, e.g. by filtration, and eluting the bound proteins from the adsorbent by suitable variation of pH and ionic strength.

Advantageously, the separation or purification process according to the present invention is carried out by interaction or chromatography, which comprises:

(a) the contact phase, wherein a mixture containing the biological material is contacted with the adsorbent comprising the compound of formula (1) retained on a chromatographic column.
(b) the washing phase, wherein the non-binding species are removed from the adsorbent comprising the compound of formula (1) by passing a washing solution there through, and
(c) the elution phase, wherein an eluting solution is passed through the adsorbent comprising the compound of formula (1) to recover the desired biological material detained from the column.

According to another aspect of the present invention there is provided an adsorbent comprising the adduct of a compound of formula (1) as defined above and a substrate having a group capable of reaction with a reactive group in said compound of formula (1) to form a covalent bond, wherein the variables are defined and of particular interest as given above.

The adsorbents according to the present invention are highly pure materials, devoid of any dye leaching behavior and contain various levels of dye-ligand concentrations on the matrix.

Furthermore, the adsorbents according to the present invention are chemically and thermally stable and highly selective in regards to binding and separation of antibodies, antibody fragments and engineered variants thereof.

The dye-ligands can be prepared analogously to procedures known in the art.

The invention will now be explained in more detail by making reference to the following examples. The temperatures are given in degrees Celsius. Unless otherwise indicated, parts are parts by weight and percentages relate to percent by weight. Parts by weight relate to parts by volume in a ratio of kilograms to litres.

## Experimental Part

### A. General Procedures

#### 1. Determination of chromatography resin performance

#### 1.1 Generation of antibody aggregates

Protein A Sepharose purified human polyclonal IgG molecules (20ml, protein content 15mg/ml) are dialyzed twice (4h and 16 respectively) against 25mM Na-acetate pH 4.0 buffer. The IgG sample is incubated at 60°C for 20h to induce the formation of IgG aggregates. The heat-treated IgG sample is subsequently adjusted to 75mM NaCl and pH 5.0 and dialyzed against buffer A1 (25mm MES 20% 1,2 propandiol pH 6.0). Untreated control IgG sample is dialyzed against buffer A1.

Figure 1 illustrates the result of an analytical SEC (Fig. 1a) and of a non-reducing SDS-PAGE analysis (Fig. 1b) (IgG = Immunoglobulin, HT = Heat treated, Mu = protein mulitmer, Mo = protein Monomer) of an IgG monomer sample and an IgG aggregate obtained by heat treatment.

The protein contents of heat-treated IgG and untreated control IgG samples are adjusted to 15mg/ml.

### 1.2 Chromatography conditions

Chromatography columns (diameter = 0.5cm, length 5,2cm) are filled with approximately 1.0ml of chromatography resin and mounted onto a standard HPLC system.

Buffers:

Loading/Equilibration buffer A1: 25mM MES pH 6.0, 20% 1,2 propandiol
Elution buffer B1: 25mM MES pH 6.0, 20% 1,2 propandiol, 1.0 M NaCl
Elution buffer B2: 25mM Tris/HCl pH8.5, 10% isopropanol
CIP buffer A2: 1.0M NaOH

Sample Application:

Sample (2.0ml, 15mg/ml) is applied with 75cm/h (0.25ml/min, retention time. 4min) and unbound protein washed away with buffer A1. Bound proteins are eluted with a linear gradient of 20CV to 100% B1 with 300cm/h (1.0ml/min) followed by complete stripping with 7 CV buffer B2 and 7 CV buffer A2. The protein sample is detected at UV 280nm and protein fractions are collected.

SDS PAGE:

10μg of sample and 15μl of 1ml fractions are loaded onto a SDS-PAGE under non-reducing conditions following Coomassie Blue staining.

Fractions of window 'A' and window 'B' are pooled, adjusted to 0.9mg/ml and dialyzed against 25mM Na-acetate pH 4.0 buffer. Equal amounts (10μg) are loaded onto SDS-PAGE under non-reducing conditions following Coomassie Blue staining.

Analytical SEC:

25μl of pooled fractions 'A' and 'B' are loaded onto a BioSep-SEC-S2000 column (Phenomenex) in running buffer (100mM Na-phosphate pH 4.0 buffer, 300mM NaCl) at 1.0ml/min at 30°C.

Isocratic protein elution is detected at UV 280nm.

### 2. Determination of the dye content (ligand density) by UV-Vis spectrophotometer

### 2.1 Preparation of the calibration curve

Preparation of the standard solution:

The dye (25 mg) is dissolved in water (80 ml) in 100 ml volumetric flask first with shaking and then using ultrasound for 10 min. The volumetric flask is filled up to the mark with water.

Preparation of the calibration solutions

Five standard solutions with concentrations of 5, 10, 15, 20 and 25 mg/l are prepared as follows. The standard solution (1, 2, 3, 4 and 5 ml) is pipetted to 50 ml volumetric flask into which HCl (6 mol/l, 40 ml) is added. The solution is mixed in the shaking bath for 1 h at 40 °C. The solution may also be mixed in the shaking bath at a higher temperature, like for 1 h at 60 °C. The solution is cooled down to room temperature and the volumetric flask is filled up to the mark with HCl (6 mol/l).

Sample preparation and measurement

In order to remove liquid from the dye sample to be analysed a conventional freeze-drying step is performed. For example, in order to dry an aqueous sample freeze-drying may be performed at -15 °C and <1 mbar reduced pressure. An aliquot of the said freeze-dried resin sample (100mg) is placed in 50 ml volumetric flask and HCl (6 mol/l, 40 ml) is added. The solution is mixed in the shaking bath for 1 h at 40 °C. The solution may also be mixed in the shaking bath at a higher temperature, like for 1 h at 60 °C. The solution is cooled down to room temperature and the volumetric flask is filled up to the mark with HCl (6 mol/l).

UV-Vis spectrum is measured using 1 cm cuvette and lamda max ($\lambda_{max}$) of particular dye. Blank setting of the instrument is done with 6M HCl.

Calculations

R = Amount of dye in the sample read from the calibration curve [mg/l]
Amount of dye in the in 50 ml volumetric flask = R / 1000 * 50 [mg]

$$\text{Ligand density} = \frac{R / 1000 * 50 * \text{purity of the dye}}{\text{Weighted sample} \times 100} \quad [mg/g]$$

**B. Synthesis examples**

**1. Preparation of dye ligands**

Dye-ligands of the following general formula 5 are synthesitzed for performing the enrichment experiments as described herein below.

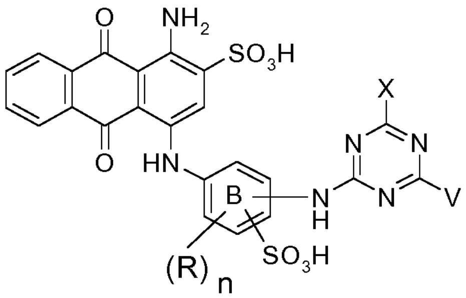

(5)

X = Halogen
R = H or methyl
n = 1, 2 or 3

Table 4

| No. | Structure Group B[1)] (including connecting NH-groups) |
|---|---|
| (B-2) | 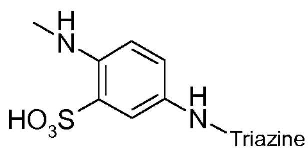 |
| (B-3) | 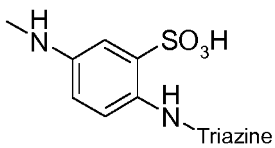 |
| (B-4) | 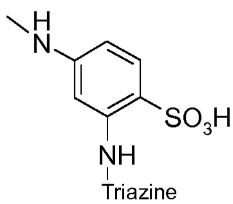 |
| 1) "Triazine" designates the linkage of the group B to the triazine ring as shown in formula 5 above | |

Table 5

| No. | Name Group V | Structure Group V[1)] |
|---|---|---|
| (V-1) | β-hydroxyethyl-amino | 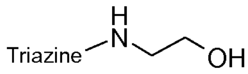 |
| (V-2) | N,N-di-β-hydroxyethyl-amino | 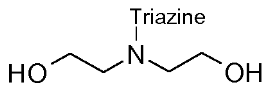 |
| (V-3) | 2-(β-hydroxyethoxy)ethylamino | 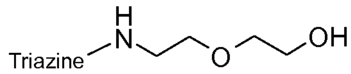 |
| (V-4) | N-ethyl-N-(m-(β-sulfatoethyl) sulfonylphenyl) | 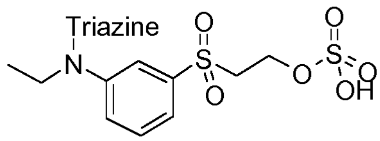 |
| (V-5) | morpholyl | 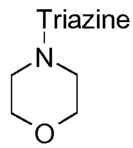 |
| (V-6) | o-sulfophenyl-amino | 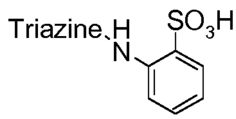 |
| (V-7) | m-sulfophenyl-amino | 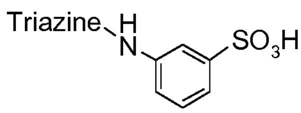 |
| (V-8) | p-sulfophenyl-amino | 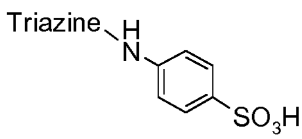 |

1) "Triazine" designates the linkage of the group V to the triazine ring as shown in formula 5 above

The particular dyes may be prepared as described in detail in WO 2006/108760.

**1.1 Preparation of the anthraquinone dyes:**

The 4-aminoaryl-substituted anthraquinone dyes can be easily prepared from the starting material bromaminic acid and the corresponding substituted phenylene diamin by Ullman coupling according to Literature (e.g. H. Zollinger, Color Chemistry, 3rd. Ed.,Wiley-VCH (2003), 273 - 278.)

**Synthesis Example D1:**

**Synthesis of Anthraquinone dye with structure group B-2:**

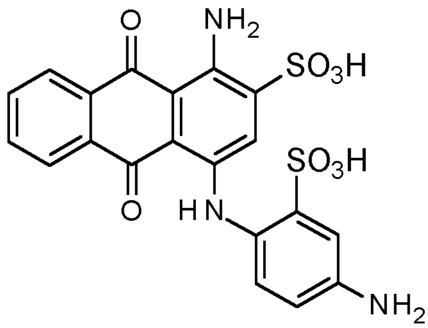

The dye was obtained by copper(I)chloride-catalyzed coupling reaction of 1-Amino-4-bromoanthraquinone-2-sulfonic acid with p-aminoacetanilide in a sodium bicarbonate buffered aqueous solution for 3 hours at 70°C, followed by sulfonation in oleum, 10 - 12% at ambient temperature, followed by hydrolysis and deprotection in diluted sulfuric acid at 75°C for 6 hours.

**Synthesis Example D2:**

**Synthesis of Anthraquinone dye with structure group B-3:**

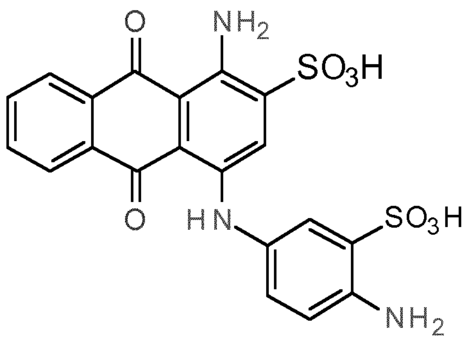

The dye was obtained by copper(II)acetate-catalyzed coupling reaction of 1-Amino-4-bromoanthraquinone-2-sulfonic acid with p-phenylenediamine sulfonic acid in a sodium carbonate buffered aqueous solution for 2 hours at 80°C.

**Synthesis Example D3:**

**Synthesis of Anthraquinone dye with structure group B-4:**

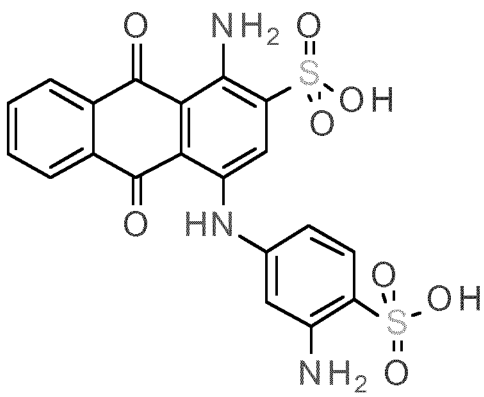

The dye was obtained by copper-catalyzed coupling reaction of 1-Amino-4-bromoanthraquinone-2-sulfonic acid with 1,3-phenylenediamine-4-sulfonic acid in a sodium bicarbonate buffered aqueous solution for 17 hours at 60°C.

**1.2 Preparation of the dye ligands:**

The dye ligands can be prepared in principle in two different ways (see also Scheme 1):

**Procedure b1)** The corresponding anthraquinone dye (B-2 to B-4) is coupled with cyanuric chloride to the blue dichloro-intermediate at pH 4 - 5 and 15°C for 90 min and subsequently coupled with the corresponding substituted amine (V-1to V-8) at pH 7.5 - 8.5 and 60°C for 5 hours, or

**Procedure b2)** The substituted amine (V-1 to V-8) is coupled with cyanuric chloride to the colorless dichloro-intermediate at pH 3.5 - 4 and 5°C for 120 min and subsequently coupled with the corresponding anthraquinone dye (B-2 to B-4) at pH 7.5 - 8.5 and 50°C for 16 hours.

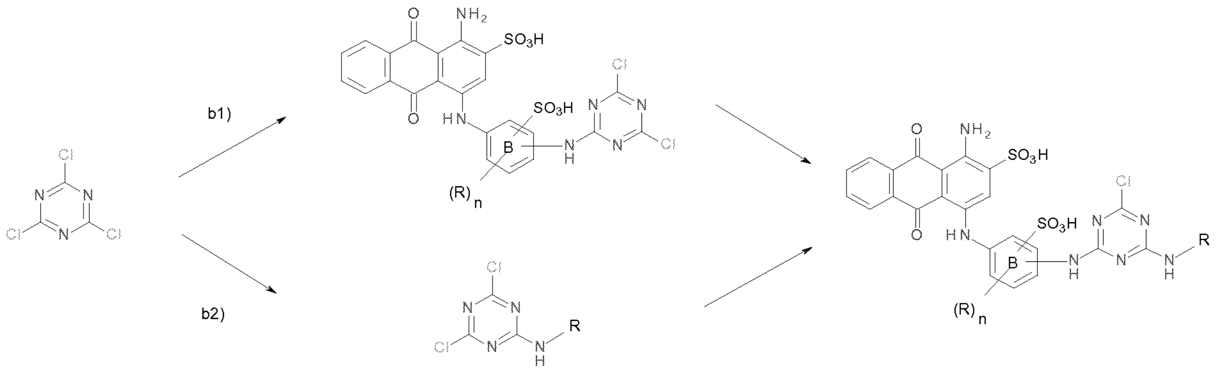


**Synthesis Example L1: Dye D15**

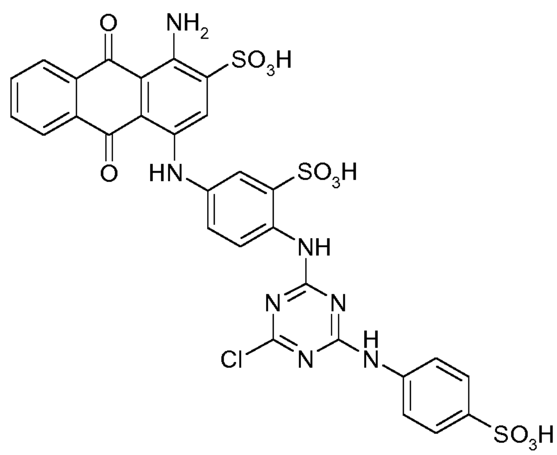

Procedure b1):

184.4 g (1.0 mol) cyanuric chloride was dissolved in 1870 ml ethyl methyl ketone at 5°C and precipitated by adding 2240 g ice water. A solution of 0,933 mol anthraquinone dye B-3 in 2240 g water and 448 g sodium carbonate solution (20%) at pH 7.5 was added to the cyanuric chloride suspension at 5°C. The reaction was allowed to warm up to 15 °C and stirred for 90 min at that temperature, while the pH value was maintained at 4 - 5 by slow addition of 112 g sodium carbonate solution (20%). After completion of the reaction (HPLC control), a solution of 194 g (1,12 mol) p-aminobenzene sulfonic acid (V-8) in 670 g water and 300 g sodium carbonate solution (20%) at pH 7.5 was added to the dichloro intermediate at 15°C. The pH was adjusted to 7.5 - 8.5 by addition of 672 g sodium carbonate solution (20%) and kept at this level until the coupling is completed. The mixture was heated to 60°C and stirred for 5 hours.

After completion of the reaction (HPLC control), 1140 g sodium chloride was added to the reaction mixture and cooled to 25°C within 2 hours and kept at this temperature for another 16 hours to precipitate the ligand dye. After filtration and washing with 1200 g of conc. sodium chloride solution, the press cake was reslurried in water and desalted by reverse osmosis. The concentrated, chloride-free dye solution was spray-dried to obtain 512 g (61% yield) of the tri-sodium salt of dye D15 as dark-blue fine powder ($\lambda_{max.}$ = 622 nm).

Procedure b2):

60.6 g (0.35 mol) p-aminobenzene sulfonic acid (V-8) was dissolved in 160 g water and 43.4 g sodium hydroxide solution (30%). In a second vessel, 65.9 g (0.36 mol) cyanuric chloride was well suspended in 370 g ice and 100 g water and the sodium p-aminobenzene sulfonate solution was added. The pH was corrected to and maintained at 2.5 - 3.5 with 420 ml sodium hydroxide solution (10%) and the temperature kept at 5°C. The reaction was stirred for 120 min, then warmed up to 40°C and stirred for another 30 min. A solution of 0.25 mol of the anthraquinone dye B-3 in 420 ml water and 150 ml sodium hydroxide solution (30%) was added quickly and the pH adjusted to and maintained at 7.5 - 8.5 with 460 ml of sodium hydroxide solution (10%) and the mixture was stirred at 50°C for 16 hours.

After completion of the reaction, 600 g of sodium chloride was added to the reaction mixture and cooled to 25°C within 2 hours and kept at this temperature for another 16 hours to precipitate the ligand dye. After filtration and washing with 530 ml of sodium chloride solution (26%), the press cake was reslurried in water and desalted by reverse osmosis. The concentrated, chloride-free dye solution was spray-dried to obtain 111 g (52% yield) of the tri-sodium salt of dye D15 as dark-blue fine powder ($\lambda_{max.}$ = 622 nm).

**Synthesis Example L2: Dye D16:**

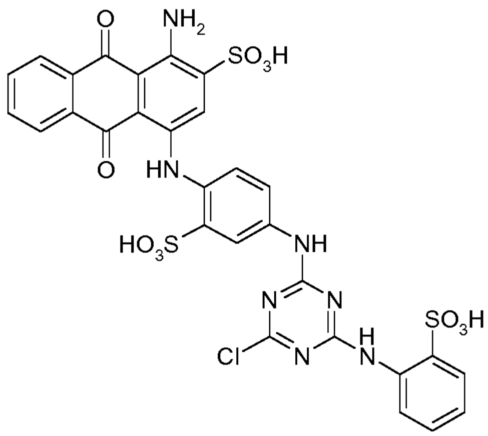

According to procedure b2) in example 1, dye D16 was obtained by coupling of 0.082 mol cyanuric chloride with 0.080 mol o-aminobenzene sulfonic acid (V-6) at pH 2.5 - 3.5 and 0 - 5°C for 120 min to obtain the dichloro intermediate and subsequent coupling of this intermediate with 0.057 mol anthraquinone dye B-2 at pH 7.5 - 8.5 and 50 °C for 16 hours in 78% yield ($\lambda_{max.}$ = 605/619 nm).

**Synthesis Example L3: Dye D11**

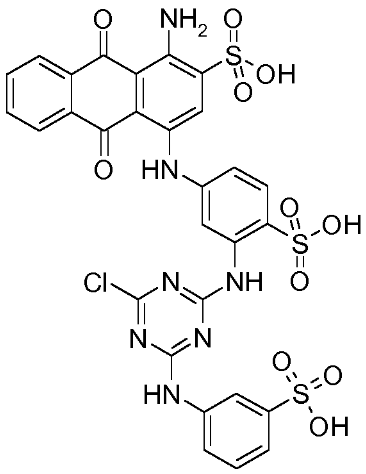

According to procedure b1) in example 1, dye D11 was obtained by coupling of 0.0315 mol cyanuric chloride with 0.030 mol anthraquinone dye B-4 at pH 4 - 5 and 15°C for 90 min to obtain the dichloro intermediate and subsequent coupling of this intermediate with 0.0318 mol m-aminobenzene sulfonic acid (V-7) at pH 7.5 - 8.5 and 60°C for 5 hours in 64% yield ($\lambda_{max.}$ = 591/624 nm).

**Synthesis Example L4: Dye D01**

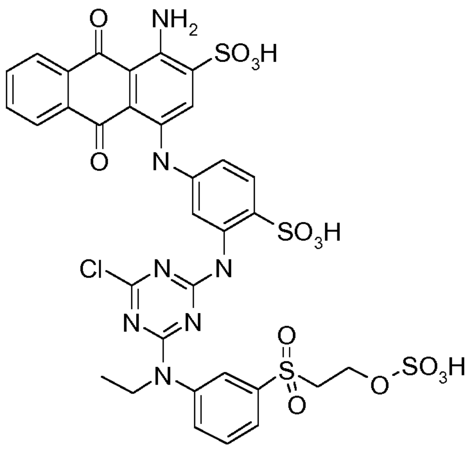

According to procedure b1) in example 1, dye D01 was obtained by coupling of 1.0 mol cyanuric chloride with 0.933 mol anthraquinone dye B-4 at pH 4 - 5 and 15°C for 90 min to obtain the dichloro intermediate and subsequent coupling of this intermediate with 1.12 mol N-ethyl-N-(m-(β-sulfatoethyl) sulfonyl-phenyl)amine (V-4) at pH 7.5 - 8.5 and 60°C for 5 hours in 49% yield ($\lambda_{max.}$ = 623 nm).

**Synthesis Example L5: Dye D21:**

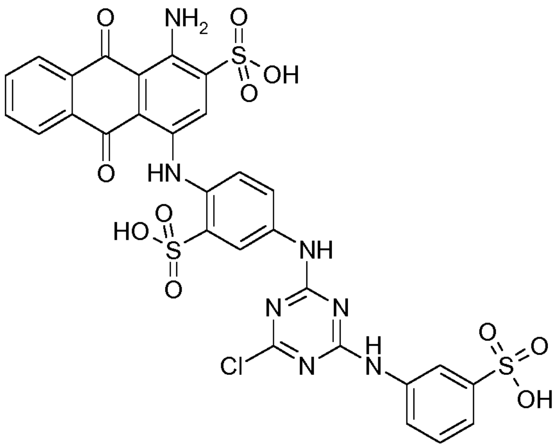

According to procedure b2) in example 1, dye D21 was obtained by coupling of 0.057 mol of cyanuric chloride with 0.056 mole of m-aminobenzene sulfonic acid (V-7) at pH 2.5 - 3.5 and 0 - 5°C for 120 min to obtain the dichloro intermediate and subsequent coupling of this intermediate with 0.040 mol of anthraquinone dye B-2 at pH 7.5 - 8.5 and 50 °C for 16 hours in 79% yield ($\lambda_{max.}$ = 609/629 nm).

**Synthesis Example L6: Dye D20:**

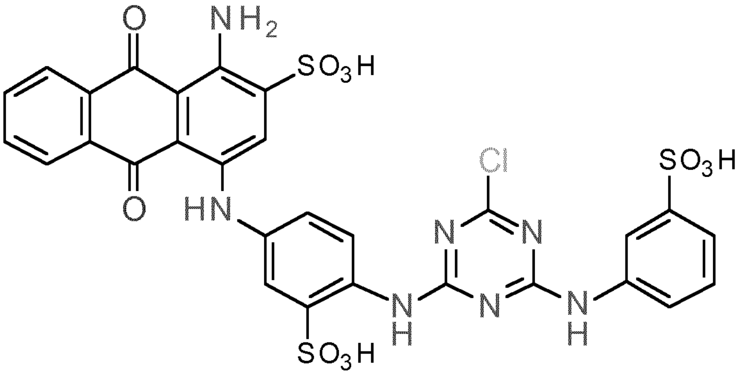

According to procedure b1) in example 1, dye D20 was obtained by coupling of 1.0 mol cyanuric chloride with 0.933 mol anthraquinone dye B-3 at pH 4 - 5 and 15°C for 90 min to obtain the dichloro intermediate and subsequent coupling of this intermediate with 1.12 mol m-aminobenzene sulfonic acid (V-7) at pH 7.5 - 8.5 and 60°C for 5 hours in 63% yield ($\lambda_{max.}$ = 627 nm).

**Synthesis Example L7: Dye D10:**

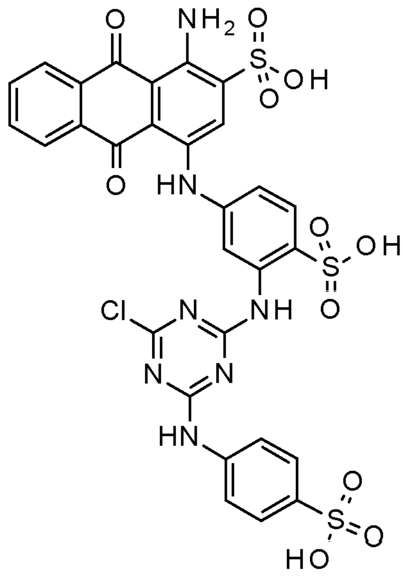

According to procedure b2) in example 1, dye D10 was obtained by coupling of 0.143 mol of cyanuric chloride with 0.140 mole of p-aminobenzene sulfonic acid (V-8) at pH 2.5 - 3.5 and 0 - 5°C for 120 min to obtain the dichloro intermediate and subsequent coupling of this intermediate with 0.100 mol of anthraquinone dye B-4 at pH 7.5 - 8.5 and 50 °C for 16 hours in 50% yield ($\lambda_{max.}$ = 595/620 nm)

**2. Preparation of Adsorbents (Resins)**

The General procedure for the synthesis of adsorbents via polyamine spacers as described in WO06/108760A2 was followed.

**2.1 Particular Adsorbents**

The following resins were prepared:

Table 6

| BASF Resin (BR) | Dye | B Group | V Group | Ligand density (μmol/g) |
|---|---|---|---|---|
| **36** | D10 | B-4 | V-8 | **284** |
| **37** | D11 | B-4 | V-7 | **281** |
| **45** | D21 | B-2 | V-7 | **280** |
| **46** | D15 | B-3 | V-8 | **307** |
| **47** | D16 | B-2 | V-6 | **256** |

The following spacer was used in these examples: 1,6-diaminohexane (DAH).

**Synthesis Example A1:**

**a) Procedure for the synthesis of an adsorbent via DAH linker - Resin BR 36, 37**

Agarose gel (6% cross-linked, 225 g) is washed with water (4500 ml) and is immediately suspended in water (900 ml) in a 2L jacketed reactor. Epichlorohydrin (37.5 g, 405 mmol) is added followed by equivalent quantity of NaOH (16.2 g, 405 mmol) and the reaction mass is shaken at 290 rpm for 4 h at 25°C. The suspension is then filtered, washed thoroughly with water (4500 ml) and drained to obtain epoxy-activated agarose.

The epoxy-activated agarose (225 g) is added to a solution of 1,6-diaminohexane (12.55 g, 108 mmol) in water (900 ml). The reaction mixture is shaken for 4 h at 45 °C and filtered. The resultant amino functionalized gel is thoroughly

washed with water (4500 ml) and drained.

The amino functionalized agarose (225 g) is divided in to 3 parts of 75 g the 1 st part is suspended in a solution of dye D10 (2.32 g, 3 mmol), in water (300 ml) and the mixture is shaken at 290 rpm for 16 h hours at 45°C. The suspension is filtered and the excess of dye-ligand is washed off with water (2000 ml), 1M NaCl (500 ml), NMP (1000 ml), 1:1 v/v NMP: water (500 mL), 1 M NaCl (300 ml), 1 M NaOH (300 ml), 1 M NaCl (300 ml) and finally water (2000 ml). The drained adsorbent BR36 is stored in 20% v/v ethanol at 4°C.

The 2nd part of amino functionalized agarose 75 g is suspended in a solution of dye D11 (2.32 g, 3 mmol), in water (300 ml) and the mixture is shaken at 290 rpm for 16 h hours at 45°C. The suspension is filtered and the excess of dye-ligand is washed off with water (2000 ml), 1M NaCl (500 ml), NMP (1000 ml), 1:1 v/v NMP: water (500 mL), 1M NaCl (300 ml), 1M NaOH (300 ml), 1M NaCl (300 ml) and finally water (2000 ml). The drained adsorbent BR37 is stored in 20% v/v ethanol at 4°C.

**b) Procedure for the synthesis of the absorbents BR45, BR46, BR47**

Agarose gel (6% cross-linked, 225 g) is washed with water (4500 ml) and is immediately suspended in water (900 ml) in a 2L jacketed reactor. Epichlorohydrin (37.5 g, 405 mmol) is added followed by equivalent quantity of NaOH (16.2 g, 405 mmol) and the reaction mass is shaken at 290 rpm for 4 h at 25°C. The suspension is then filtered, washed thoroughly with water (4500 ml) and drained to obtain epoxy-activated agarose.

The epoxy-activated agarose (225 g) is added to a solution of 1,6-diaminohexane (12.55 g, 108 mmol) in water (900 ml). The reaction mixture is shaken for 4 h at 45 °C and filtered. The resultant amino functionalized gel is thoroughly washed with water (4500 ml) and drained.

The amino functionalized agarose (225 g) is divided in to 3 parts of 75 g the 1 st part is suspended in a solution of dye D21 (2.32 g, 3 mmol), in water (300 ml) and the mixture is shaken at 290 rpm for 16 h hours at 45°C. The suspension is filtered and the excess of dye-ligand is washed off with water (2000 ml), 1M NaCl (500 ml), NMP (1000 ml), 1:1 v/v NMP: water (500 mL), 1M NaCl (300 ml), 1M NaOH (300 ml), 1M NaCl (300 ml) and finally water (2000 ml). The drained adsorbent B45 is stored in 20% v/v ethanol at 4°C.

The 2nd part of amino functionalized agarose 75 g is suspended in a solution of dye D19 (2.32 g, 3 mmol), in water (300 ml) and the mixture is shaken at 290 rpm for 16 h hours at 45°C. The suspension is filtered and the excess of dye-ligand is washed off with water (2000 ml), 1M NaCl (500 ml), NMP (1000 ml), 1:1 v/v NMP: water (500 mL), 1M NaCl (300 ml), 1M NaOH (300 ml), 1M NaCl (300 ml) and finally water (2000 ml). The drained adsorbent BR46 is stored in 20% v/v ethanol at 4°C.

The 3rd part of amino functionalized agarose 75 g is suspended in a solution of dye D16 (2.32 g, 3 mmol), in water (300 ml) and the mixture is shaken at 290 rpm for 16 h hours at 45°C. The suspension is filtered and the excess of dye-ligand is washed off with water (2000 ml), 1M NaCl (500 ml), NMP (1000 ml), 1:1 v/v NMP: water (500 mL), 1M NaCl (300 ml), 1M NaOH (300 ml), 1M NaCl (300 ml) and finally water (2000 ml). The drained adsorbent BR47 is stored in 20% v/v ethanol at 4°C.

**C. Test Examples**

**Example T1: Insufficient separation of HMWA (high molecular weight aggregates) from monomeric IgG molecules (prior art)**

IgG antibody aggregates are prepared as described in the methods section (A.1.1).

An aggregate sample was analysed on a commercial Blue Sepharose ® (BS) resin (Dye ligand density 137 μmol/g) as described above under A.1.2. In particular, the column eluate was pooled as depicted in Figure 2a. Pools A (BS-A) and B (BS-B) were further analyzed by SDS-PAGE (Figure 2b) and analytical size exclusion chromatography (Figure 2c).

(L = Load, Mu = protein multimer, Mo = protein monomer; non reducing SDS-PAGE with Coomassie Brilliant blue staining) An insufficient separation of Mo and Mu in particular in pool B is evident.

**Example T2: Improved separation of HMWA from monomeric IgG molecules by applying a BASF resins of the present invention**

IgG antibody aggregates are prepared as described in the methods section (A.1.1).

An aggregate sample was analysed on BASF resins BR36, BR37, BR45, BR46 and BR47 (Dyle ligands D10, D11, D21, D15, D16 with Dye ligand densities 284, 281, 280, 307, 256 μmol/g respectively; C6 linker) as described above under A.1.2 (see Figures 3a and 3b). Single fractions of each chromatographic separation were analyzed by SDS-PAGE (Figure 4) indicating improved aggregrate (Mu) removal. To further analyze superior separation characteristics of BASF resins, the column eluate was pooled as depicted in Figure 2a and 3a,b. Pools A and B were further analyzed by analytical size exclusion chromatography (Figure 5) and by SDS PAGE (Figure 6)
(L = Load, FT = Flowthrough, Mu = protein multimer, Mo = protein monomer; non reducing SDS-PAGE with Coomassie Brilliant blue staining)
An improved separation of Mo and Mu, in particular in pool B, if compared to commercial Blue sepharose (Example T1) is evident.

## Claims

**1.** An adsorbent for chromatographic purification of immunoglobulin molecules, which adsorbent comprises a carrier matrix to which a dye ligand is covalently attached, wherein said dye ligand is selected from anthraquinone dyes and wherein the ligand density of said adsorbent is more than 150 μmoles/g dry adsorbent.

**2.** The adsorbent of claim 1, wherein the ligand density of said adsorbent is 160 to 500 μmoles/g dry adsorbent, preferably 200 to 350 μmoles/g dry adsorbent.

**3.** The adsorbent of claim 1 or 2, wherein said adsorbent comprises the reaction product of said carrier matrix, optionally a linker molecule and an anthraquinone compound of the general formula 1

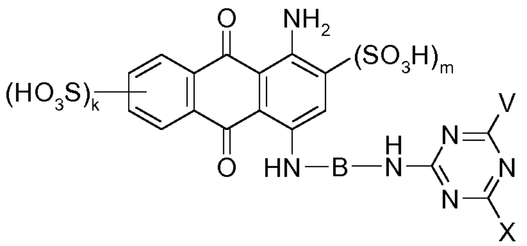

(1)

wherein

X is halogen;
k and m are each independently of each other 0 or 1 and the sum of k+m being 1 or 2;
B is an aromatic radical, comprising one or more, condensed or non-condensed, optionally mono- or polysubstituted aromatic rings;
V is a substituent of formula

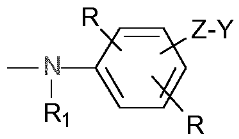

(2a)

or

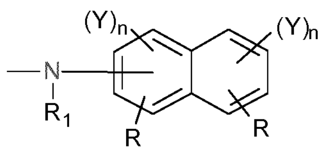

(2b)

wherein

n is each 0 or 1; with the proviso that both n are not simultaneously 0;
R are the same or different and are selected from H, alkyl, $NH_2$, NH-alkyl, NH-aryl, $N(alkyl)_2$, $NO_2$, OH, O-alkyl, CN, C(O)alkyl, C(O)aryl, $C(O)NH_2$, C(O)N(H)alkyl, $C(O)N(alkyl)_2$, C(O)N(H)aryl, halogen
$R_1$ is hydrogen or optionally substituted $C_1$-$C_4$alkyl;
Z is selected from a chemical bond,

$-(CH_2)_{n1}-$ with n1 being an integer from 1 to 4,
arylene,
$-CH_2$-arylene-,
$-SO_2$-arylene-,
-C(O)N(H)arylene-,
$-SO_2N(H)$arylene-,
-CH=CH-,
$-SO_2-CH_2-CH_2-$;
$-C(O)NH-(CH_2)_{n2}-$ with n2 being an integer from 1 to 4;
and
Y are the same or different polar functional groups selected from $-SO_3H$,$-OSO_3H$, $-CO_2H$, $-P(O)(OH)_2$, $-OP(O)(OH)_2$, OH and SH.

**4.** The adsorbent of anyone of the preceding claims, wherein
B is a substituent of formula

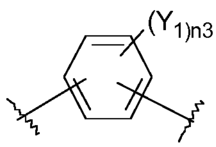

(3a)

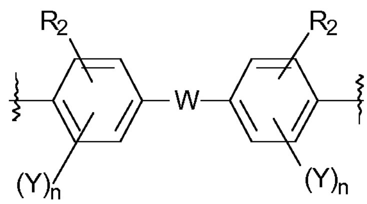

(3b)

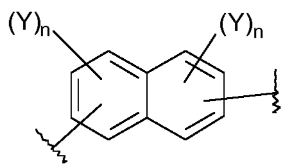

(3c)

or

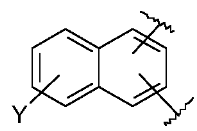

(3d)

wherein

Y is as defined above,
n is 0 or 1
n3 is 0, 1, 2, 3 or 4
$Y_1$ are the same or different and are selected from halogen, alkyl, alkoxy, $NO_2$ or Y, wherein Y is as defined above;
W is a chemical bond or is selected from -C(O)N(H)-, $-SO_2N(H)-$, $-SO_2-$ or -N(H)-; and
$R_2$ is selected from H or alkyl.

**5.** The adsorbent of anyone of the preceding claims, wherein B is an aromatic radical of formula 3a, and/or wherein V is a substituent for formula 2a.

**6.** The adsorbent of claim 5, wherein B is an aromatic radical of formula 3a, wherein n3 is 0 , 1, 2, 3 or 4 and $Y_1$ is

independently alkyl or Y.

7. The adsorbent of claim 5 wherein V is a substituent for Formula 2a, wherein R is H and Z is selected from a chemical bond or $-SO_2-CH_2-CH_2-$.

8. The adsorbent of anyone of the preceding claims wherein k is 0 and m is 1.

9. The adsorbent of anyone of the claims 1 to 8, having a mean particle size in the range of 10 to 200, like 20 to 180 or 45 to 165 or 80 to 120 or 90 to 110 μm.

10. A process for chromatographic purification of immunoglobulin molecules, which process comprises contacting a sample containing at least one desired type of immunoglobulin molecules in admixture with contaminating inorganic and/or organic substances with an adsorbent of anyone of the claims 1 to 9, adsorbing said desired type of immunoglobulin molecules on said adsorbent, optionally washing said adsorbent with a washing liquid, and afterwards eluting the adsorbed material in order to obtain an eluate containing said desired immunoglobulin molecules in enriched form; or which process comprises contacting a sample containing at least one desired type of immunoglobulin molecules in a mixture with contaminating inorganic and/or organic substances with an adsorbent of anyone of the claims 1 to 9, adsorbing said contaminating inorganic and/or organic substances, obtaining a flowthrough containing said desired immunoglobulin molecules in a more pure form.

11. The process of claim 10, wherein said desired type of immunoglobulin molecule is a functional antibody (Ab) or fragment antibody (fAb)

12. The process of one of the claims 10 and 11, wherein the immunoglobulin containing sample to be purified is contaminated with denatured and/or agglomerated /mulitimerized Immunoglobulins.

13. The use of a compound as defined in any one of the claims 1 to 8 for the separation of immunoglobulin molecules, in particular Abs and fAbs.

14. A preparative or analytical kit comprising at least one adsorbent carrying an anthraquinone compound as defined in any one of the claims 1 to 9, and additional material for identifying at least one separated fAb.

15. A method of preparing an adsorbent of one of the claim 1 to 9, which method comprises reacting an optionally activated carrier with a suitable linker, as for example tri-2'-aminoethylamine (TREN), followed by reacting said thus obtained functionalized adsorbent with an anthraquinone compound of claim 1 to 8.

## Patentansprüche

1. Adsorptionsmittel zur chromatographischen Reinigung von Immunglobulinmolekülen, wobei das Adsorptionsmittel eine Trägermatrix umfasst, an die ein Farbstoffligand kovalent gebunden ist, wobei der Farbstoffligand ausgewählt ist aus Anthrachinonfarbstoffen und wobei die Ligandendichte des Adsorptionsmittels mehr als 150 μmol/g trockenes Adsorptionsmittel beträgt.

2. Adsorptionsmittel nach Anspruch 1, wobei die Ligandendichte des Adsorptionsmittels 160 bis 500 μmol/g trockenes Adsorptionsmittel, vorzugsweise 200 bis 350 μmol/g trockenes Adsorptionsmittel, beträgt.

3. Adsorptionsmittel nach Anspruch 1 oder 2, wobei das Absorptionsmittel das Reaktionsprodukt der Trägermatrix, gegebenenfalls ein Linkermolekül und eine Anthrachinonverbindung der allgemeinen Formel (1) umfasst

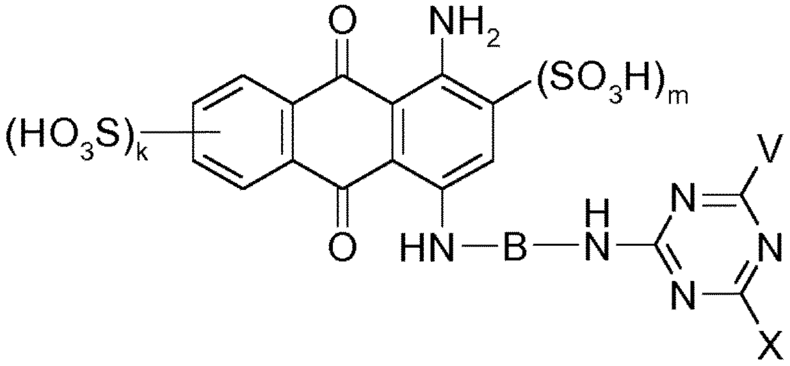

(1)

worin

X für Halogen steht;
k und m jeweils unabhängig voneinander für 0 oder 1 stehen und die Summe von k + m 1 oder 2 ist;
B für einen aromatischen Rest steht, der einen oder mehrere kondensierte oder nicht kondensierte, gegebenenfalls einfach oder mehrfach substituierte aromatische Ringe umfasst;
V für einen Substituenten der Formel

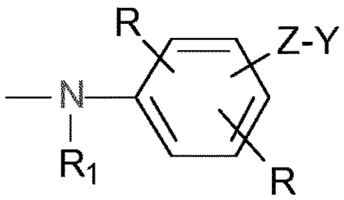

(2a)

oder

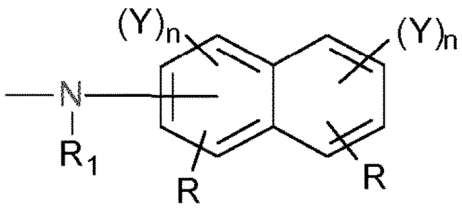

(2b)

steht, worin
n jeweils für 0 oder 1 steht; mit der Maßgabe, dass beide n nicht gleichzeitig 0 sind;
R gleich oder verschieden sind, und ausgewählt sind aus H, Alkyl, $NH_2$, NH-Alkyl, NH-Aryl, $N(Alkyl)_2$, $NO_2$, OH, O-Alkyl, CN, C(O)Alkyl, C(O)Aryl, $C(O)NH_2$, C(O)N(H)Alkyl, $C(O)N(Alkyl)_2$, C(O)N(H)Aryl, Halogen
$R_1$ für Wasserstoff oder gegebenenfalls substituiertes $C_1$-$C_4$Alkyl steht;
Z ausgewählt ist aus einer chemischen Bindung,
$-(CH_2)_{n1}-$ wobei n1 für eine ganze Zahl von 1 bis 4 steht,
Arylen,
$-CH_2$-Arylen-,
$-SO_2$-Arylen-,
-C(O)N(H)Arylen-,
$-SO_2N(H)$Arylen-,
-CH=CH-,
$-SO_2-CH_2-CH_2-$;
$-C(O)NH-(CH_2)_{n2}-$, wobei n2 für eine ganze Zahl von 1 bis 4 steht;
und
Y für gleiche oder verschiedene polare funktionelle Gruppen stehen, ausgewählt aus $-SO_3H$, $-OSO_3H$, $-CO_2H$, $-P(O)(OH)_2$, $-OP(O)(OH)_2$, OH und SH.

4. Adsorptionsmittel nach einem der vorhergehenden Ansprüche, wobei B für einen Substituenten der Formel

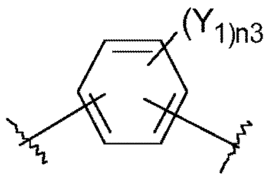

(3a)

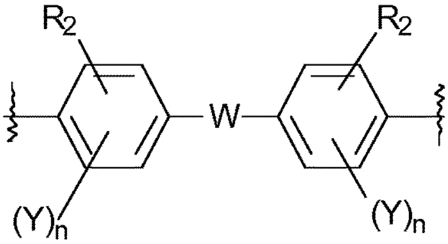

(3b)

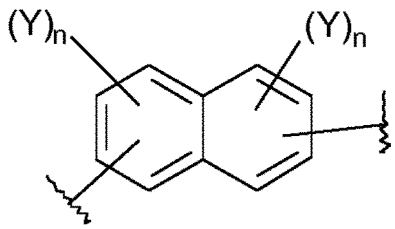

(3c)

oder

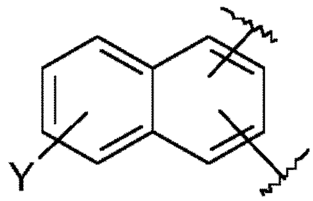

(3d)

steht, worin

Y wie oben definiert ist,
n für 0 oder 1 steht
n3 für 0, 1, 2, 3 oder 4 steht,
$Y_1$ gleich oder verschieden sind und ausgewählt sind aus Halogen, Alkyl, Alkoxy, $NO_2$ oder Y, wobei Y wie oben definiert ist;
W für eine chemische Bindung steht oder ausgewählt ist aus -C(O)N(H)-, $-SO_2N(H)-$, $-SO_2-$ oder -N(H)-; und
$R_2$ ausgewählt ist aus H oder Alkyl.

5. Adsorptionsmittel nach einem der vorhergehenden Ansprüche, wobei B für einen aromatischen Rest der Formel 3a steht, und/oder wobei V für einen Substituenten für Formel 2a steht.

6. Adsorptionsmittel nach Anspruch 5, wobei B für einen aromatischen Rest der Formel 3a steht, worin n3 für 0, 1, 2, 3 oder 4 steht und $Y_1$ unabhängig für Alkyl oder Y steht.

7. Adsorptionsmittel nach Anspruch 5, wobei V für einen Substituenten für Formel 2a steht, worin R für H steht und Z ausgewählt ist aus einer chemischen Bindung oder $-SO_2-CH_2-CH_2-$.

8. Adsorptionsmittel nach einem der vorhergehenden Ansprüche, wobei k für 0 steht und m für 1 steht.

**9.** Adsorptionsmittel nach einem der Ansprüche 1 bis 8, das eine mittlere Teilchengröße im Bereich von 10 bis 200, wie 20 bis 180 oder 45 bis 165 oder 80 bis 120 oder 90 bis 110 μm aufweist.

**10.** Verfahren zur chromatographischen Reinigung von Immunglobulinmolekülen, wobei das Verfahren das Inkontaktbringen einer Probe, die mindestens einen gewünschten Typ von Immunglobulinmolekülen im Gemisch mit kontaminierenden anorganischen und/oder organischen Substanzen enthält, mit einem Adsorptionsmittel nach einem der Ansprüche 1 bis 9, das Adsorbieren des gewünschten Typs von Immunglobulinmolekülen auf dem Adsorptionsmittel, gegebenenfalls das Waschen des Adsorptionsmittels mit einer Waschflüssigkeit und anschließend das Eluieren des adsorbierten Materials umfasst, um ein Eluat zu erhalten, das die gewünschten Immunglobulinmoleküle in angereicherter Form enthält; oder wobei das Verfahren das Inkontaktbringen einer Probe, die mindestens einen gewünschten Typ von Immunglobulinmolekülen im Gemisch mit kontaminierenden anorganischen und/oder organischen Substanzen enthält, mit einem Adsorptionsmittel nach einem der Ansprüche 1 bis 9, das Adsorbieren der kontaminierenden anorganischen und/oder organischen Substanzen umfasst, wodurch ein Durchfluss erhalten wird, der die gewünschten Immunglobulinmoleküle in einer reineren Form enthält.

**11.** Verfahren nach Anspruch 10, wobei der gewünschte Typ von Immunglobulinmolekül ein funktioneller Antikörper (Ab) oder Fragment-Antikörper (fAb) ist.

**12.** Verfahren nach einem der Ansprüche 10 und 11, wobei die zu reinigende Immunglobulin-enthaltende Probe mit denaturierten und/oder agglomerierten/multimerisierten Immunglobulinen kontaminiert ist.

**13.** Verwendung einer Verbindung nach einem der Ansprüche 1 bis 8 für die Trennung von Immunglobulinmolekülen, insbesondere Abs und fAbs.

**14.** Präparatives oder analytisches Kit, umfassend mindestens ein Adsorptionsmittel, das eine Anthrachinonverbindung nach einem der Ansprüche 1 bis 9 trägt, und zusätzliches Material zum Identifizieren von mindestens einem getrennten fAb.

**15.** Verfahren zur Herstellung eines Adsorptionsmittel nach einem der Anspruch 1 bis 9, wobei das Verfahren das Umsetzen eines gegebenenfalls aktivierten Trägers mit einem geeigneten Linker, wie zum Beispiel tri-2'-Aminoethylamin (TREN), gefolgt vom Umsetzen des auf diese Weise erhaltenen funktionalisierten Adsorptionsmittels mit einer Anthrachinonverbindung nach Anspruch 1 bis 8 umfasst.

## Revendications

**1.** Adsorbant pour purification chromatographique de molécules d'immunoglobuline, ledit adsorbant comprenant une matrice de support à laquelle un ligand colorant est lié de façon covalente, dans lequel ledit ligand colorant est choisi parmi des colorants anthraquinones et dans lequel la densité de ligand dudit adsorbant est supérieure à 150 μmoles/g d'adsorbant sec.

**2.** Adsorbant de la revendication 1, dans lequel la densité de ligand dudit adsorbant est de 160 à 500 μmoles/g d'adsorbant sec, de préférence de 200 à 350 μmoles/g d'adsorbant sec.

**3.** Adsorbant de la revendication 1 ou 2, ledit adsorbant comprenant le produit de réaction de ladite matrice de support, facultativement une molécule de lieur et un composé d'anthraquinone de formule générale

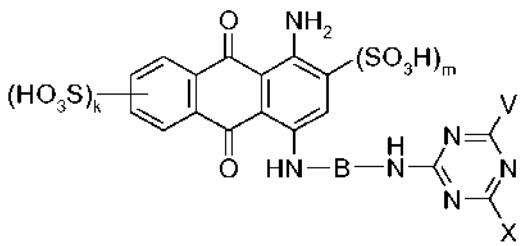

(1)

dans laquelle

X est halogène ;
k et m sont chacun indépendamment l'un de l'autre 0 ou 1 et la somme de k+m étant 1 ou 2 ;
B est un radical aromatique, comprenant un ou plusieurs cycles aromatiques, condensés ou non condensés, facultativement mono- ou polysubstitués ;
V est un substituant de formule

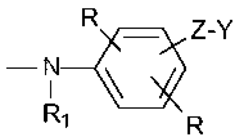

(2a)

ou

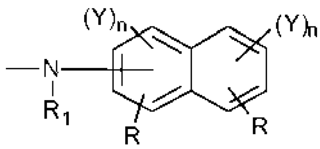

(2b)

dans laquelle
n est chacun 0 ou 1 ; à condition que les deux n ne soient pas simultanément 0 ;
R sont identiques ou différents et sont choisis parmi H, alkyle, $NH_2$, NH-alkyle, NH-aryle, $N(alkyle)_2$, $NO_2$, OH, O-alkyle, CN, C(O)alkyle, C(O)aryle, $C(O)NH_2$, C(O)N(H)alkyle, $C(O)N(alkyle)_2$, C(O)N(H)aryle, halogène $R_1$ est hydrogène ou alkyle en $C_1$-$C_4$ facultativement substitué ;
Z est choisi parmi une liaison chimique,
$-(CH_2)_{n1}-$ avec n1 étant un entier de 1 à 4, arylène,
$-CH_2$-arylène-,
$-SO_2$-arylène-,
-C(O)N(H)arylène-,
$-SO_2N(H)$arylène-,
-CH=CH-,
$-SO_2-CH_2-CH_2-$ ;
$-C(O)NH-(CH_2)_{n2}-$ avec n2 étant un entier de 1 à 4 ; et
Y sont des groupes polaires identiques ou différents choisis parmi $-SO_3H$, $-OSO_3H$, $-CO_2H$, $-P(O)(OH)_2$, $-OP(O)(OH)_2$, OH et SH.

**4.** Adsorbant de l'une quelconque des revendications précédentes, dans lequel
B est un substituant de formule

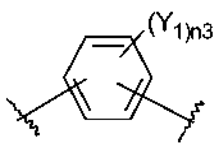

(3a)

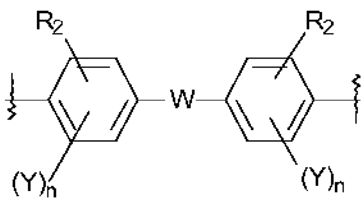

(3b)

ou

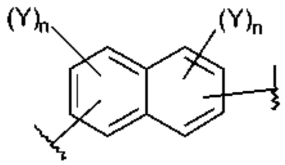

(3c)

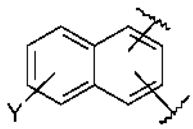

(3d)

dans lequel

Y est tel que défini ci-dessus,
n est 0 ou 1
n3 est 0, 1, 2, 3 ou 4
$Y_1$ sont identiques ou différents et sont choisis parmi halogène, alkyle, alcoxy, $NO_2$ ou Y, où Y est tel que défini ci-dessus ;
W est une liaison chimique ou est choisi parmi -C(O)N(H)-, $-SO_2N(H)-$, $-SO_2-$ ou -N(H)- ; et
$R_2$ est choisi parmi H ou alkyle.

5. Adsorbant de l'une quelconque des revendications précédentes, dans lequel B est un radical aromatique de formule 3a, et/ou dans lequel V est un substituant pour la formule 2a.

6. Adsorbant de la revendication 5, dans lequel B est un radical aromatique de formule 3a, dans lequel n3 est 0, 1, 2, 3 ou 4 et $Y_1$ est indépendamment alkyle ou Y.

7. Adsorbant de la revendication 5 dans lequel V est un substituant pour la formule 2a, dans lequel R est H et Z est choisi parmi une liaison chimique ou $-SO_2-CH_2-CH_2-$.

8. Adsorbant de l'une quelconque des revendications précédentes dans lequel k est 0 et m est 1.

9. Adsorbant de l'une quelconque des revendications 1 à 8, ayant une taille de particule moyenne dans la plage de 10 à 200, telle que 20 à 180 ou 45 à 165 ou 80 à 120 ou 90 à 110 $\mu$m.

10. Procédé de purification chromatographique de molécules d'immunoglobuline, ledit procédé comprenant la mise en contact d'un échantillon contenant au moins un type souhaité de molécules d'immunoglobuline en mélange avec des substances contaminantes inorganiques et/ou organiques avec un adsorbant de l'une quelconque des revendications 1 à 9, l'adsorption dudit type souhaité de molécules d'immunoglobuline sur ledit adsorbant, facultativement le lavage dudit adsorbant avec un liquide de lavage, et ensuite l'élution du matériau adsorbé afin d'obtenir un éluat contenant lesdites molécules d'immunoglobuline souhaitées sous forme enrichie ; ou ledit procédé comprend la mise en contact d'un échantillon contenant au moins un type souhaité de molécules d'immunoglobuline dans un mélange avec des substances contaminantes inorganiques et/ou organiques avec un adsorbant de l'une quelconque des revendications 1 à 9, l'adsorption desdites substances contaminantes inorganiques et/ou organiques, l'obtention d'un flux continu contenant lesdites molécules d'immunoglobuline souhaitées sous une forme plus pure.

11. Procédé de la revendication 10, dans lequel ledit type souhaité de molécule d'immunoglobuline est un anticorps (Ab) ou un fragment d'anticorps (fAb) fonctionnel.

12. Procédé de l'une des revendications 10 et 11, dans lequel l'échantillon contenant des immunoglobulines devant être purifié est contaminé par des immunoglobulines dénaturées et/ou agglomérées / multimérisées.

13. Utilisation d'un composé tel que défini dans l'une quelconque des revendications 1 à 8 pour la séparation de molécules d'immunoglobuline, en particulier des Ab et des fAb.

14. Kit préparatif ou analytique comprenant au moins un adsorbant comportant un composé d'anthraquinone tel que

défini dans l'une quelconque des revendications 1 à 9, et un matériau additionnel pour identifier au moins un fAb séparé.

**15.** Procédé de préparation d'un adsorbant de l'une des revendications 1 à 9, ledit procédé comprenant la réaction d'un support facultativement activé avec un lieur adapté, tel que, par exemple, la tri-2'-aminoéthylamine (TREN), suivie par la réaction dudit adsorbant fonctionnalisé obtenu ainsi avec un composé d'anthraquinone des revendications 1 à 8.

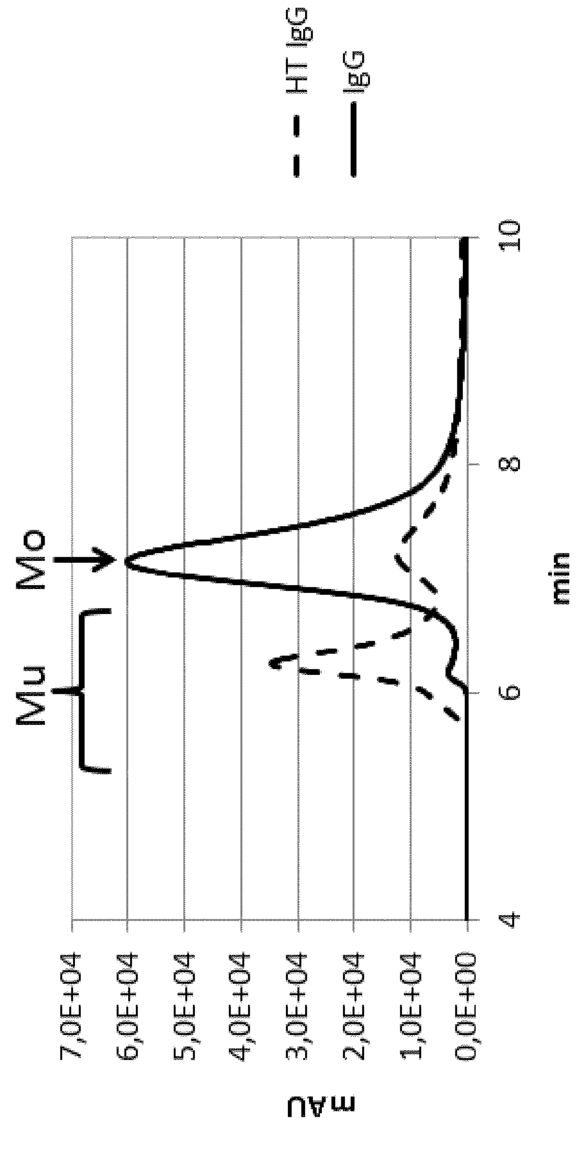

mAU
7,0E+04
6,0E+04
5,0E+04
4,0E+04
3,0E+04
2,0E+04
1,0E+04
0,0E+00
4
6
8
10
min
Mu
Mo
HT IgG
IgG


Fig. 1a

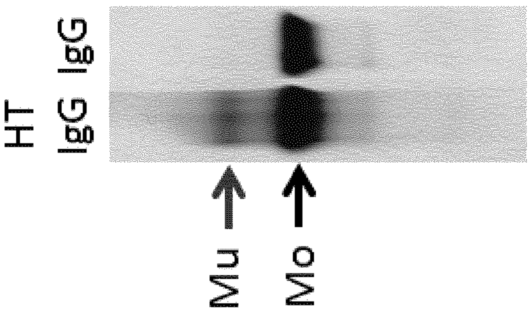

HT IgG
IgG
Mu
Mo


Fig. 1b

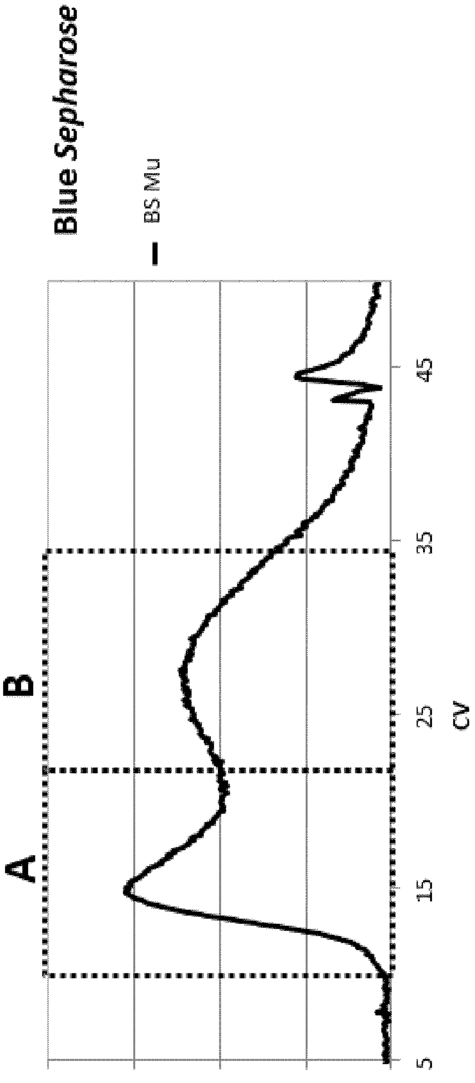


Fig. 2a

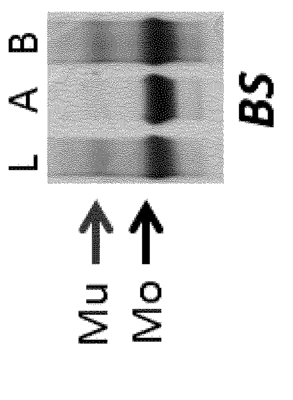


Fig. 2b

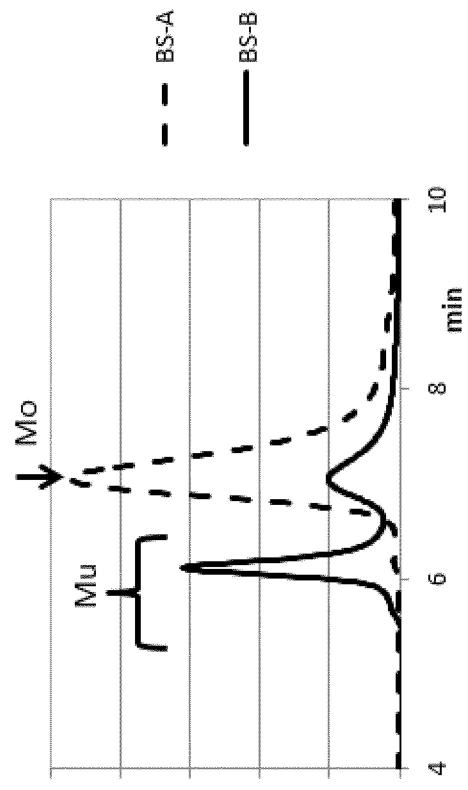


Fig. 2c

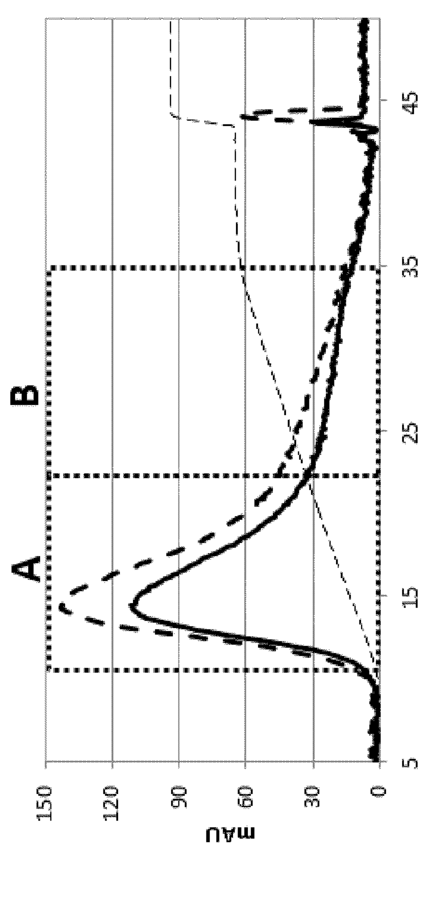
BR36 Mu
BR37 Mu
mS/cm
A
B
mAU
150
120
90
60
30
0
5
15
25
35
45
CV

Fig. 3a

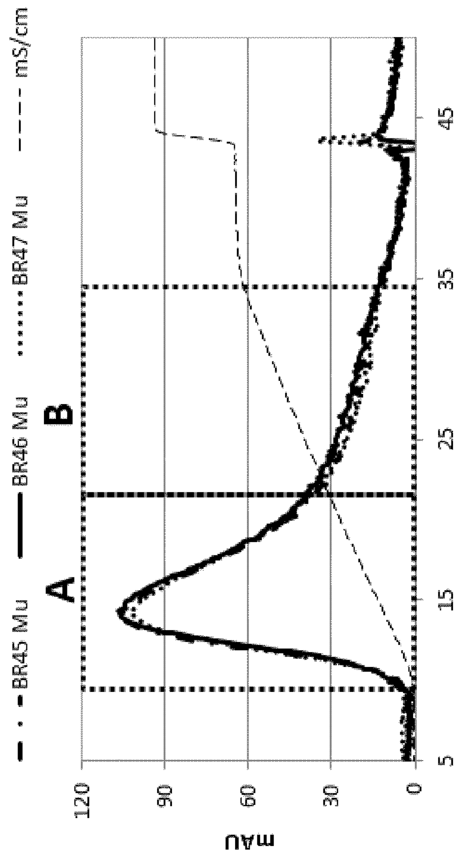
BR45 Mu
BR46 Mu
BR47 Mu
mS/cm
A
B
mAU
120
90
60
30
0
5
15
25
35
45
CV

Fig.3b

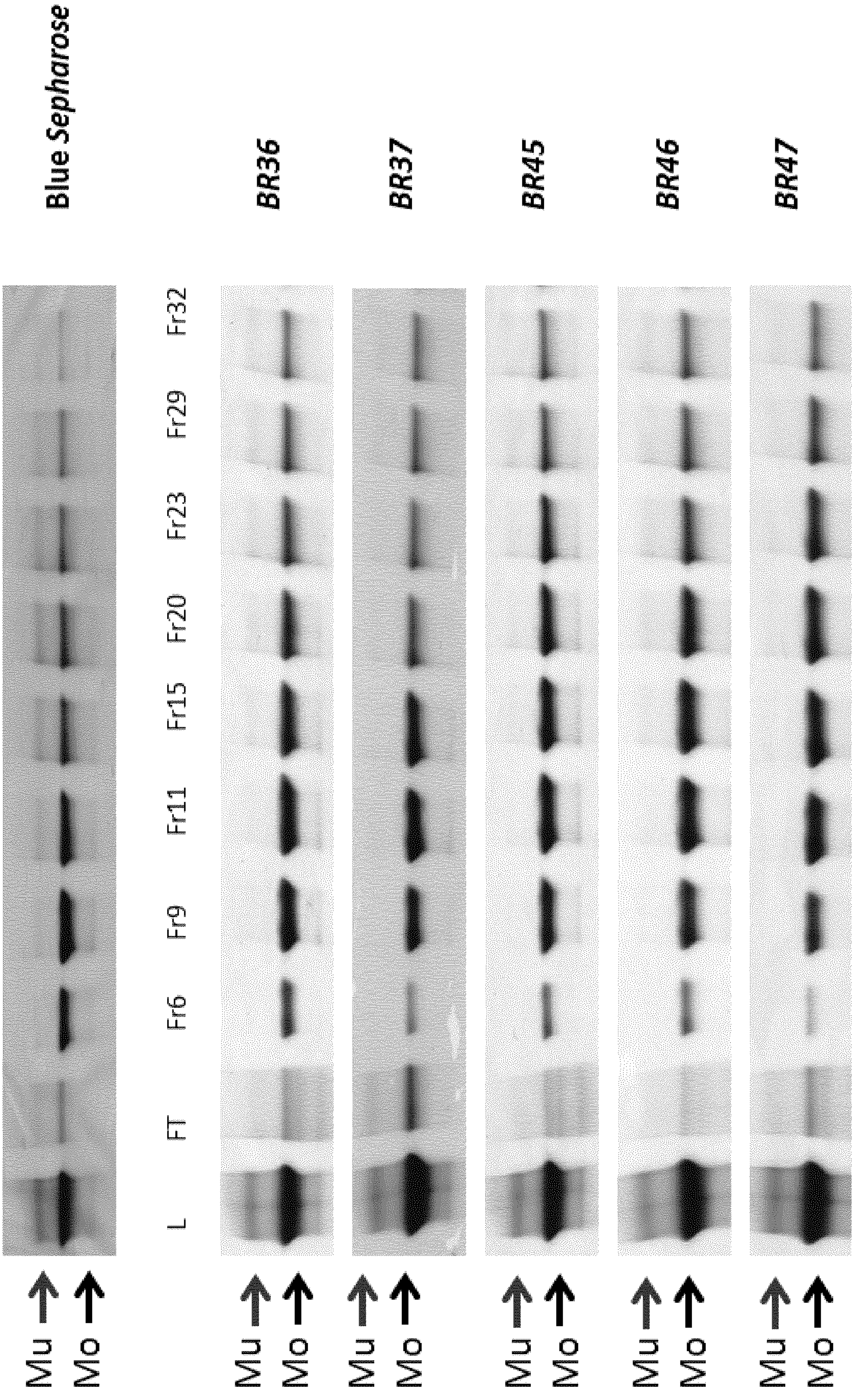
Blue Sepharose
BR36
BR37
BR45
BR46
BR47
L
FT
Fr6
Fr9
Fr11
Fr15
Fr20
Fr23
Fr29
Fr32
Mu
Mo

Fig. 4

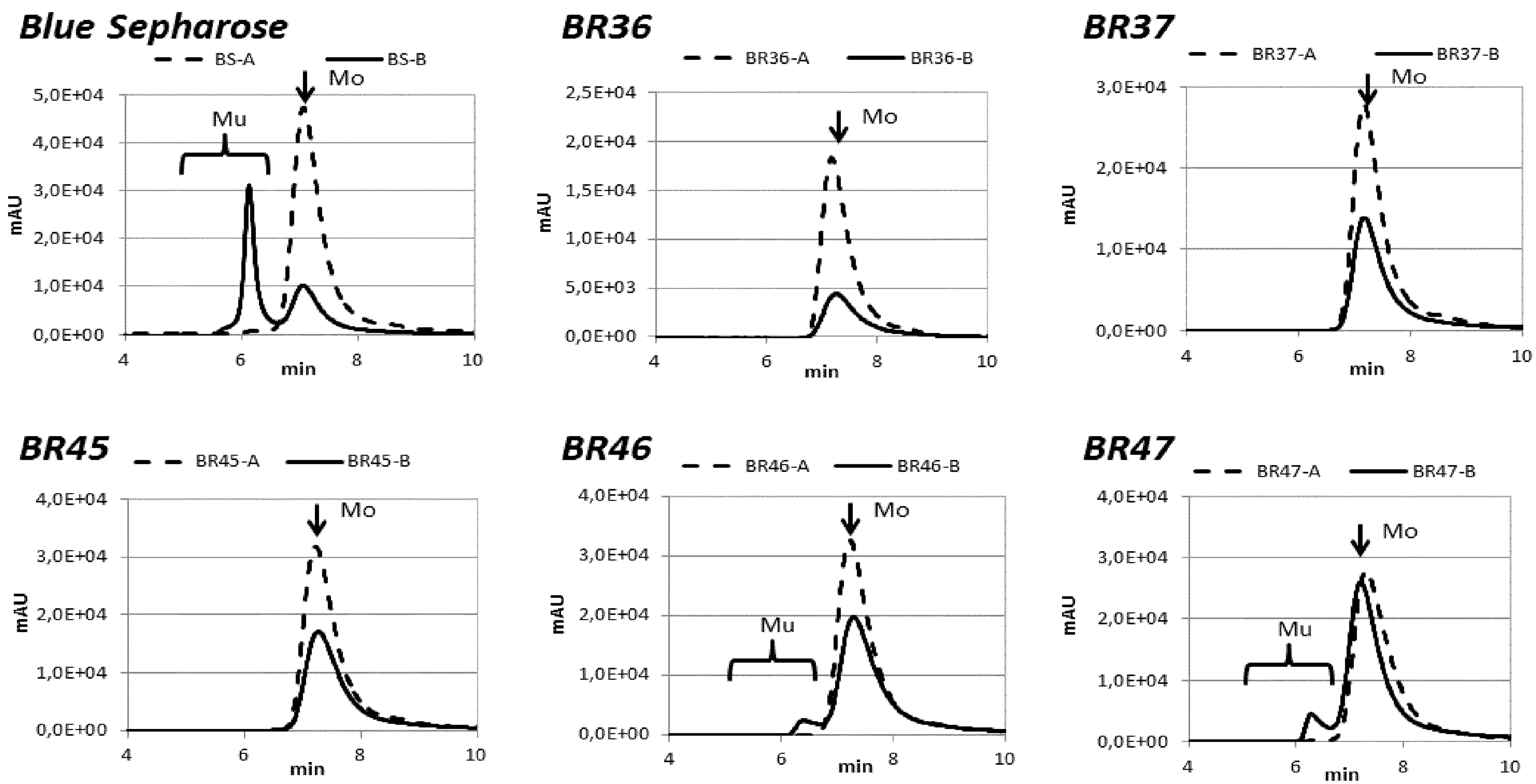
Blue Sepharose
BS-A
BS-B
Mo
Mu
5,0E+04
4,0E+04
3,0E+04
2,0E+04
1,0E+04
0,0E+00
mAU
4
6
8
10
min
BR36
BR36-A
BR36-B
Mo
2,5E+04
2,0E+04
1,5E+04
1,0E+04
5,0E+03
0,0E+00
BR37
BR37-A
BR37-B
Mo
3,0E+04
2,0E+04
1,0E+04
0,0E+00
BR45
BR45-A
BR45-B
Mo
4,0E+04
3,0E+04
2,0E+04
1,0E+04
0,0E+00
BR46
BR46-A
BR46-B
Mo
Mu
BR47
BR47-A
BR47-B
Mo
Mu

**Fig. 5**

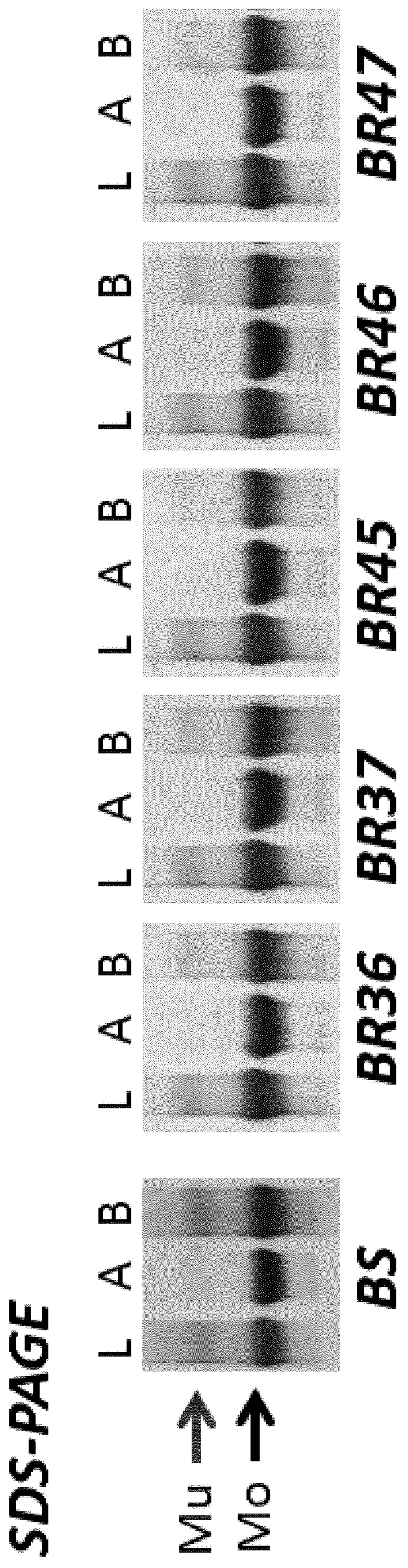
SDS-PAGE
Mu
Mo
L A B
BS
L A B
BR36
L A B
BR37
L A B
BR45
L A B
BR46
L A B
BR47

Fig. 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4016149 A
- US 4546161 A
- WO 2004052870 A
- WO 2006108760 A
- WO 9710887 A
- WO 2004035199 A
- WO 2007099374 A
- WO 2007004954 A
- WO 2009138714 A
- WO 2010102114 A
- GB 1540165 A
- WO 06108760 A2


### Non-patent literature cited in the description


- Affinity Chromatography: A Practical Approach. 1982
- **HOLLINGER et al.** *Nature Biotechnology,* 2005, vol. 23 (9), 1126-1136
- **H. ZOLLINGER.** Color Chemistry. Wiley-VCH, 2003, 273-278